# EUROPEAN PATENT SPECIFICATION

(11) **EP 3 274 318 B1**
(45) Date of publication and mention of the grant of the patent: **26.02.2020**
(21) Application number: 15823117.5
(22) Date of filing: 21.12.2015
(51) Int. Cl.: C07C 2/74, C07C 5/367, C07C 13/28, C07C 15/14

(54) **PRODUCTION OF BIPHENYL COMPOUNDS**
HERSTELLUNG VON BIPHENYLVERBINDUNGEN
PRODUCTION DE COMPOSÉS BIPHÉNYLE

(30) Priority: 25.03.2015 US 201562137996 P; 09.06.2015 EP 15171178
(43) Date of publication of application: 31.01.2018
(73) Proprietor: ExxonMobil Chemical Patents Inc., Baytown, TX 77520 (US)
(72) Inventor: SALCICCIOLI, Michael, Houston, TX 77021 (US); DAKKA, Jihad M., Whitehouse Station, NJ 08889 (US); SANGAR, Neeraj, League City, TX 77573 (US); DECAUL, Lorenzo C., Langhorne, PA 19047 (US); KHEIR, Ali A., Houston, TX 77004-2832 (US)
(74) Representative: ExxonMobil Chemical Europe Inc.
(86) International application number: PCT/US2015/067112
(87) International publication number: WO 2016/153575

(56) References cited:
- US-A1- 2014 275 609

## Description

### FIELD OF THE INVENTION

The disclosure relates to methyl-substituted biphenyl compounds, their production and their use in the manufacture of plasticizers.

### BACKGROUND OF THE INVENTION

Plasticizers are incorporated into a resin (usually a plastic or elastomer) to increase the flexibility, workability, or distensibility of the resin. The largest use of plasticizers is in the production of "plasticized" or flexible polyvinyl chloride (PVC) products. Typical uses of plasticized PVC include films, sheets, tubing, coated fabrics, wire and cable insulation and jacketing, toys, flooring materials such as vinyl sheet flooring or vinyl floor tiles, adhesives, sealants, inks, and medical products such as blood bags and tubing, and the like.

Other polymer systems that use small amounts of plasticizers include polyvinyl butyral, acrylic polymers, nylon, polyolefins, polyurethanes, and certain fluoroplastics. Plasticizers can also be used with rubber (although often these materials fall under the definition of extenders for rubber rather than plasticizers). A listing of the major plasticizers and their compatibilities with different polymer systems is provided in "Plasticizers," A. D. Godwin, in Applied Polymer Science 21st Century, edited by C. D. Craver and C. E. Carraher, Elsevier (2000); pp. 157-175.

The most important chemical class of plasticizers is phthalic acid esters, which accounted for about 84% worldwide of PVC plasticizer usage in 2009. However, there is an effort to decrease the use of phthalate esters as plasticizers in PVC, particularly in end uses where the product contacts food, such as bottle cap liners and sealants, medical and food films, or for medical examination gloves, blood bags, and IV delivery systems, flexible tubing, or for toys, and the like. As a result, there is a need for non-phthalate, mono- or diester plasticizers, particularly oxo-ester plasticizers, that can be made from low cost feeds and employ few manufacturing steps in order to have comparable economics with their phthalate counterparts.

To this end, suggested substitutes for phthalates recently have included biphenylester-based plasticizers. For instance, U.S. Patent Publication No. 2014/0275609 teaches, among other things, the manufacture of dimethylbiphenyl compounds containing significant amounts of the 3,3'-dimethyl, the 3,4'-dimethyl and the 4,4'-dimethyl isomers. Such compounds can be economically produced by hydroalkylation of toluene and/or xylene followed by dehydrogenation of the resulting (methylcyclohexyl)toluene and/or (dimethylcyclohexyl)xylene product. As also taught in U.S. Patent Publication No. 2014/0275609, the resultant mixture can be used as a precursor in the production of biphenylester-based plasticizers by, for example, oxidizing the methyl-substituted biphenyl compounds to convert at least one of the methyl groups to a carboxylic acid group and then esterifying the carboxylic acid group with an alcohol, such as an oxo alcohol.

The proposed synthesis of dimethylbiphenyl compounds includes a first step of hydroalkylation of an aromatic hydrocarbon (e.g., benzene, toluene, and/or xylene, among others), which produces desired phenylcyclohexane and/or alkylated phenylcyclohexane intermediates, as well as undesired cyclohexane and/or alkylcyclohexane intermediate byproducts. These intermediates and intermediate byproducts can be dehydrogenated to form, respectively, (i) the desired biphenyl and/or dialkylbiphenyl product (e.g., dimethylbiphenyl) and (ii) benzene and/or alkylated benzene (e.g., toluene). Commercially desirable efficiency is achieved by separating and recycling the benzene and/or alkylated benzene for utilization as additional hydroalkylation feed.

The present inventors have found that the efficiency of the process can surprisingly be further improved by an additional, separate dehydrogenation of the cyclohexane and/or alkylcyclohexane intermediate byproducts in the absence of C₁₂ or greater hydrocarbons to provide benzene and/or alkylated benzene for the additional hydroalkylation feed.

Additional references of interest may include U.S. Patent Nos. 6,730,625 and 6,037,513; U.S. Patent Publication Nos. 2014/0275605, 2014/0275606, 2014/0275607, 2014/0323782; Sinfelt, J. Mol. Cat. A., 163 (2000), at 123; and Sinfelt et al., J. Phys. Chem., 64 (1960), at 1559.

### SUMMARY OF THE INVENTION

The present invention is concerned with processes as defined in claim 1 and 12. In one aspect, the present disclosure is directed to a process for producing biphenyl compounds. The process includes hydroalkylation of a feed comprising a Cₙ aromatic hydrocarbon and hydrogen, where n may be from 6 to 12. The feed may include a mixture of C₆ - C₁₂ aromatic hydrocarbons, in which case the "Cₙ aromatic hydrocarbon" refers to the smallest (least number of carbon atoms) C₆ - C₁₂ aromatic hydrocarbon in the feed. Suitable C₆ - C₁₂ aromatic hydrocarbons include benzene, C₇ - C₁₁ alkyl-substituted benzenes (such as toluene, xylene, ethylbenzene, and/or diethylbenzene), and C₁₀ - C₁₂ naphthalenes (e.g., naphthalene and/or methyl, dimethyl, and/or ethyl-naphthalenes).

The hydroalkylation is carried out in the presence of a hydroalkylation catalyst under conditions effective to produce a hydroalkylation reaction effluent. The hydroalkylation comprises stepwise hydrogenation and alkylation, such that a portion of the Cₙ aromatic hydrocarbons are partially hydrogenated to corresponding Cₙ cyclic olefin intermediates, which in turn react *in situ* with a further portion of the Cₙ aromatic hydrocarbons to form one or more C₂ₙ cycloalkylaromatic compounds. In addition, some of the Cₙ aromatic hydrocarbons are completely hydrogenated (e.g., toluene may be completely dehydrogenated to methylcyclohexane). The hydroalkylation effluent therefore comprises (i) aC₂ₙ cycloalkylaromatic compound and (ii) a Cₙ saturated cyclic hydrocarbon, in addition to any unreacted Cₙ aromatic hydrocarbon feed. In certain aspects where n ranges from 6 to 12, the C₂ₙ cycloalkylaromatic compound is therefore a C₁₂ - C₂₄ compound. For instance, where the Cₙ aromatic hydrocarbon of the feed is toluene, the C₂ₙ cycloalkylaromatic compound is (methylcyclohexyl)toluene. Where C₆ - C₁₂ aromatic hydrocarbons other than the Cₙ aromatic hydrocarbons are present in the hydroalkylation feed, such C₆ - C₁₂ aromatic hydrocarbons similarly undergo hydroalkylation to corresponding C₁₂ - C₂₄ cycloalkylaromatic compounds.

Both the cycloalkylaromatic compounds and the saturated cyclic hydrocarbons are thereafter dehydrogenated. In particular, (i) the C₂ₙ cycloalkylaromatic compounds (and any other C₁₂ - C₂₄ cycloalkylaromatic compounds) are dehydrogenated to one or more biphenyl compounds, the desired product; and (ii) the Cₙ saturated cyclic hydrocarbons (and any other C₆ - C₁₂ saturated cyclic hydrocarbons) are dehydrogenated to the corresponding aromatic hydrocarbons, which may advantageously be recycled to provide additional hydroalkylation feed.

Because both the cycloalkylaromatic compounds and the saturated cyclic hydrocarbons in the hydroalkylation effluent are dehydrogenated, it would conventionally be thought that both species should be dehydrogenated together in a single dehydrogenation zone, or in multiple dehydrogenation zones in series, without substantial separation of the cycloalkylaromatic compounds from the saturated cyclic hydrocarbons.

However, the present inventors have discovered that, counterintuitively, at least a portion of the saturated cyclic hydrocarbons should be dehydrogenated separately from the cycloalkylaromatic compounds.

Without wishing to be bound by theory, it is believed that the presence of higher hydrocarbons surprisingly reduces the conversion rate of the Cₙ saturated cyclic hydrocarbon in the dehydrogenation reaction. Thus, a separation should be made so as to divide the smallest of the hydroalkylation product cycloalkylaromatic compounds from the saturated cyclic hydrocarbons. Thus, where a hydroalkylation feed comprises C₆ - C₁₂ aromatic hydrocarbons, including a Cₙ aromatic hydrocarbon which is the smallest (least number of carbon atoms) among the C₆ - C₁₂ aromatic hydrocarbons, the separation should be made so as to provide (i) a light stream rich in the Cₙ aromatic hydrocarbons, and (ii) a heavy stream rich in C₂ₙ (and heavier) cycloalkylaromatic compounds. That is, the dehydrogenation of saturated cyclic hydrocarbons should take place separately from any C₂ₙ or greater cycloalkylaromatic compound.

Therefore, the process according to some aspects further includes providing at least a portion of the hydroalkylation reaction effluent (comprising Cₙ saturated cyclic hydrocarbons, and C₂ₙ cycloalkylaromatic compounds) to a first dehydrogenation zone, and therein dehydrogenating at least a portion of the C₂ₙ cycloalkylaromatic compound and at least a portion of the Cₙ saturated cyclic hydrocarbon in the presence of a first dehydrogenation catalyst under conditions effective to produce a first dehydrogenation reaction product comprising (i) a mixture of C₂ₙ biphenyl compounds; (ii) a recovered Cₙ aromatic hydrocarbon; and (iii) unreacted Cₙ saturated cyclic hydrocarbon. At least a portion of the first dehydrogenation reaction product is thereafter separated into (i) a heavy dehydrogenation stream rich in the C₂ₙ biphenyl compounds, and (ii) a light dehydrogenation stream rich in the recovered Cₙ aromatic hydrocarbon and the unreacted Cₙ saturated cyclic hydrocarbon, and further depleted in the C₂ₙ biphenyl compounds. At least a portion of the light dehydrogenation stream is provided to a second dehydrogenation zone, wherein the unreacted Cₙ saturated cyclic hydrocarbon in said portion of the light dehydrogenation stream is dehydrogenated to obtain additional recovered Cₙ aromatic hydrocarbon. The recovered Cₙ aromatic hydrocarbons may be recycled to provide additional hydroalkylation feed.

Alternatively, in other aspects, the process may include separation of the hydroalkylation reaction effluent, e.g., prior to any dehydrogenation. In such aspects, the process includes separating the hydroalkylation reaction effluent into (i) a heavy hydroalkylation effluent rich in the C₂ₙ cycloalkylaromatic compound, and (ii) a light hydroalkylation effluent rich in the Cₙ saturated cyclic hydrocarbon, and depleted in the C₂ₙ cycloalkylaromatic compound. The process further includes providing at least a portion of the heavy hydroalkylation effluent to a first dehydrogenation zone, and therein dehydrogenating at least a portion of the C₂ₙ cycloalkylaromatic compound in the presence of a first dehydrogenation catalyst under conditions effective to produce a heavy dehydrogenation reaction product comprising a mixture of C₂ₙ biphenyl compounds. The light hydroalkylation effluent is provided to a second dehydrogenation zone, and therein at least a portion of the Cₙ saturated cyclic hydrocarbon is dehydrogenated in the presence of a second dehydrogenation catalyst under conditions effective to produce a light dehydrogenation reaction product comprising a recovered Cₙ aromatic hydrocarbon. The recovered Cₙ aromatic hydrocarbon may be recycled to provide additional hydroalkylation feed.

### BRIEF DESCRIPTION OF THE DRAWINGS

Figure 1 is a simplified process flow diagram illustrating a biphenyl compound production process in accordance with some aspects of the present invention.
Figure 2 is a simplified process flow diagram illustrating another biphenyl compound production process in accordance with some aspects of the present invention.
Figure 3 is a graph of methylcyclohexane (MCH) and (methylcyclohexyl)toluene (MCHT) conversion against weight hourly space velocity (WHSV) in the dehydrogenation of MCH and MCHT according to Example 2.
Figures 4a and 4b are graphs of MCH and MCHT conversion, respectively, against time on stream (TOS) in the dehydrogenation of MCH and MCHT according to Example 3.
Figure 5 is a graph of MCH conversion against time on stream (TOS) in the dehydrogenation of MCH according to Example 4.
Figure 6 is a graph of MCH conversion against time on stream in the dehydrogenation of MCH in the presence of toluene, according to Example 5.

### DETAILED DESCRIPTION OF THE EMBODIMENTS

The present disclosure relates to the production of biphenyl compounds by the catalytic hydroalkylation of Cₙ aromatic hydrocarbons such as C₆ - C₁₂ aromatic hydrocarbons (e.g., benzene, toluene, xylene, ethylbenzene, diethylbenzene, and the like). Depending on the catalyst employed in the hydroalkylation reaction, the hydroalkylation process is selective to the production of the desired C₂ₙ cycloalkylaromatic compounds, which, depending on the aromatic hydrocarbon fed to the hydroalkylation process, may include any one or more of various C₁₂ - C₂₄ cycloalkylaromatic compounds (e.g., phenylcyclohexane (also known as cyclohexylbenzene), (methylcyclohexyl)toluene, (dimethylcyclohexyl)xylene, (ethylcyclohexyl) ethylbenzene, and (diethylcyclohexyl) diethylbenzene. Nonetheless, fully saturated rings will also be produced as byproducts, such as Cₙ (e.g., C₆ - C₁₂ saturated cyclic hydrocarbons such as cyclohexane, methylcyclohexane, dimethylcyclohexane, ethylcyclohexane, and/or diethylcyclohexane, also depending upon the aromatic hydrocarbons present in the feed). The desired C₂ₙ cycloalkylaromatic compounds are dehydrogenated to form a mixture of biphenyl compounds, such as biphenyl, and/or methyl-, ethyl- or other alkyl- substituted biphenyl compounds. According to some embodiments, the mixture of biphenyl compounds includes dimethylbiphenyls (e.g., where the C₇ aromatic hydrocarbon toluene is present in the hydroalkylation feed). Such dimethylbiphenyl product of the dehydrogenation reaction contains significant amounts of the 3,3'-dimethyl, the 3,4'-dimethyl and the 4,4'-dimethyl compounds, making the product according to such embodiments an attractive precursor in the production of biphenylester-based plasticizers.

The byproduct Cₙ saturated cyclic hydrocarbons are advantageously also dehydrogenated to recover Cₙ aromatic hydrocarbons, which may be recycled to provide additional hydroalkylation feed. It is possible (and conventionally would be considered desirable) to conduct this additional dehydrogenation together with the dehydrogenation of the C₂ₙ cycloalkylaromatic compounds. However, the present inventors have discovered that substantial improvements in the conversion rate of Cₙ saturated cyclic hydrocarbons to Cₙ aromatic hydrocarbons are realized with dehydrogenation of the Cₙ saturated cyclic hydrocarbons separately from C₂ₙ or greater cycloalkylaromatic compounds resulting from hydroalkylation of the Cₙ aromatic hydrocarbon feed.

### Definitions

As used herein, the numbering scheme for the Periodic Table Groups is as disclosed in Chemical and Engineering News, 63(5), 27 (1985).

As used herein, a "Cₓ hydrocarbon," where x is an integer, refers to a hydrocarbon compound having X carbon atoms. Thus, a C₆ hydrocarbon is a hydrocarbon having 6 carbon atoms. A "Cₓ - C_{y} hydrocarbon" is a hydrocarbon having from x to y carbon atoms (e.g., a C₆ - C₁₀ hydrocarbon is a hydrocarbon having 6, 7, 8, 9, or 10 carbon atoms); a "Cₓ or greater" hydrocarbon is a hydrocarbon having x or more carbon atoms; and a "greater than Cₓ hydrocarbon" is a hydrocarbon having more than x carbon atoms. Similarly, a "Cₓ or less" hydrocarbon is one having x or fewer carbon atoms, and "a less than Cₓ" hydrocarbon is one having fewer than x carbon atoms.

At certain points herein, reference is made to various "Cₙ" compounds. In such instances, unless otherwise indicated, n may be an integer ranging from 6 to 12, inclusive. Along these lines, a "C₂ₙ" hydrocarbon is therefore a hydrocarbon having 2^{∗}n carbon atoms, with reference to a Cₙ hydrocarbon, and a "C₃ₙ" hydrocarbon is similarly a hydrocarbon having 3^{∗}n carbon atoms. For instance, where a Cₙ hydrocarbon is given as a C₇ hydrocarbon (e.g., toluene), a C₂ₙ hydrocarbon would be a C₁₄ hydrocarbon. Where a mixture of multiple species of C₆ - C₁₂ hydrocarbons is referred to, the Cₙ hydrocarbon will be the smallest (least number of carbon atoms) among those C₆ - C₁₂ hydrocarbons (thus, the mixture may be referred to as being of, e.g., Cₙ - C₁₂ hydrocarbons). Similarly, where a mixture of C₁₂ - C₂₄ hydrocarbons is referred to, the C₂ₙ hydrocarbon will be the smallest (least number of carbon atoms) among those C₁₂ - C₂₄ hydrocarbons. Relatedly, then, a Cₙ₊₁ hydrocarbon may refer to a hydrocarbon having 1 more carbon atom than the Cₙ hydrocarbon (such that a Cₙ - C₁₂ mixture may comprise a Cₙ hydrocarbon and one or more Cₙ₊₁ - C₁₂ hydrocarbons).

An "aromatic hydrocarbon" is a hydrocarbon containing an aromatic ring compound, and includes alkyl-substituted aromatic ring compounds. For instance, a C₆ aromatic hydrocarbon is an aromatic ring-containing hydrocarbon having 6 carbon atoms, such as benzene. Similarly, a C₇ aromatic hydrocarbon refers to a hydrocarbon compound containing an aromatic ring and having 7 carbon atoms, such as toluene. Thus, a "C₆ - C₁₂ aromatic hydrocarbon," for example, is a hydrocarbon having 6-12 carbon atoms and containing an aromatic ring. Such hydrocarbons include, but are not necessarily limited to: benzene, toluene, ethylbenzene, xylene, diethylbenzene, propylbenzene, methylpropylbenzene, butylbenzene, and alkyl naphthalenes.

Along similar lines, a "saturated cyclic hydrocarbon" is a saturated hydrocarbon containing a carbon ring moiety. Thus, cyclohexane is an example of a C₆ saturated cyclic hydrocarbon. Similarly, methylcyclohexane is an example of a C₇ saturated cyclic hydrocarbon, and (dimethyl)cyclohexane and ethylcyclohexane are both examples of C₈ saturated cyclic hydrocarbons.

Similarly, a "C₁₂ - C₂₀ cycloalkylaromatic compound" may refer to any of phenylcyclohexane (a C₁₂ hydrocarbon, also known as cyclohexylbenzene) and C₁₃ - C₂₄ substituted phenylcyclohexanes containing a substituted moiety in place of one or more hydrogens on either the phenyl or cyclohexane moiety. Particularly contemplated C₁₃ - C₂₄ substituted phenylcyclohexanes include alkyl-substituted phenylcyclohexanes (i.e., those containing one or more alkyl groups substituted in place of one or more hydrogens, such as (methylcyclohexyl)toluene, (dimethylcyclohexyl)xylene, or the like). As used herein, "alkyl-substituted" means one or more hydrogen atoms in the hydrocarbon is replaced by an alkyl moiety, such as methyl, ethyl, propyl, butyl, etc. In particular embodiments, the alkyl substitution may have from 1 to 10 carbon atoms, and in certain embodiments, from 1 to 5 carbon atoms. Also contemplated are C₁₆ - C₂₄ cycloalkylaromatic compounds comprising a naphthyl and/or decalin moiety, such as cyclohexylnapthlalene (a C₁₆ substituted cycloalkylaromatic compound comprising a naphthyl moiety), and/or naphthyldecalin (a C₂₀ cycloalkylaromatic compound including both a naphthyl and decalin moiety). Either or both rings of such fused-ring structures may further contain an alkyl substitution.

As used herein, "biphenyl compounds" refer to biphenyl and/or substituted biphenyls. Thus, a C₁₂ - C₂₄ biphenyl compound is a biphenyl or substituted biphenyl compound having 12-24 carbon atoms. Particularly contemplated are alkyl-substituted biphenyls, examples of which include biphenyl, dimethylbiphenyl, diethylbiphenyl, tetramethylbiphenyl, tetraethylbiphenyl, and so forth. Further, as with the cycloalkylaromatic compounds discussed above, also contemplated within this definition are biphenyl compounds in which either or both phenyl ring is substituted with a fused phenyl ring, such as in the case of a binaphthyl or alkyl-substituted binaphthyl compound.

### Hydroalkylation of Cₙ aromatic hydrocarbons

Hydroalkylation is a two-stage catalytic reaction in which an aromatic compound is partially hydrogenated to produce a cyclic olefin intermediate, which then reacts, *in situ,* with the aromatic compound to produce a cycloalkylaromatic product. In the present process, the aromatic compound comprises a Cₙ aromatic hydrocarbon (preferably a C₆ - C₁₂ aromatic hydrocarbon), and the cycloalkylaromatic product comprises one or more C₂ₙ (preferably C₁₂ - C₂₄) cycloalkylaromatic compounds.

In some embodiments, the Cₙ aromatic hydrocarbon is a C₆ - C₁₂ aromatic hydrocarbon, such as benzene, a C₇ - C₁₁ alkyl benzene, or a C₁₀ - C₁₂ naphthalene or alkyl-substituted naphthalene. In certain embodiments, the C₇ - C₁₁ alkyl benzene is selected from the group consisting of toluene, ethylbenzene, xylene, and diethylbenzene, with toluene and/or xylene being particularly preferred in certain embodiments. In particular embodiments, the Cₙ aromatic hydrocarbon in the hydroalkylation feed is toluene or xylene. In some embodiments, the hydroalkylation feed may comprise multiple species of aromatic hydrocarbons (e.g., a mixture of toluene and xylenes). In such cases, as noted previously, the "Cₙ aromatic hydrocarbon" refers to the smallest (lowest number of carbons) aromatic hydrocarbon. Thus, in the example given where toluene and xylene are present in the feed, n = 7 (corresponding to toluene). Accordingly, the hydroalkylation feed of some embodiments may comprise one or more Cₙ - C₁₂ aromatic hydrocarbons, where n ranges from 6 to 12, and is the smallest species of aromatic hydrocarbon in the hydroalkylation feed.

Furthermore, it will be appreciated that in some preferred embodiments, where the hydroalkylation feed comprises a C₆ aromatic hydrocarbon (e.g., benzene), it is preferable that the hydroalkylation feed not contain a C₁₂ aromatic hydrocarbon (e.g., an alkyl naphthalene, or the like). This is because such C₁₂ aromatic hydrocarbons may be difficult to separate, by conventional means (e.g., fractionation), from the C₁₂ cycloalkylaromatic compounds that will result from hydroalkylation of the C₆ aromatic hydrocarbon. Further, as discussed elsewhere herein, Cₙ aromatic hydrocarbon will form byproduct Cₙ saturated cyclic hydrocarbon byproducts during hydroalkylation, which should be dehydrogenated at least in part separately from any C₂ₙ compounds. Of course, where n = 6, this means dehydrogenation separately from C₁₂ compounds. Therefore, any C₁₂ aromatic hydrocarbons converted to C₁₂ cyclic hydrocarbon byproduct along with the C₆ cyclic hydrocarbon byproducts would not be retained in the C₆ cut of the separation, for further dehydrogenation and recycling. Such C₁₂ byproducts would therefore be significantly more difficult to recover for recycling.

A Cₙ aromatic hydrocarbon will hydroalkylate to a desired corresponding C₂ₙ species by the aforementioned stepwise hydrogenation and alkylation process, as shown in the overall reaction scheme illustrated below for the example of toluene (C₇ aromatic hydrocarbon) feed:

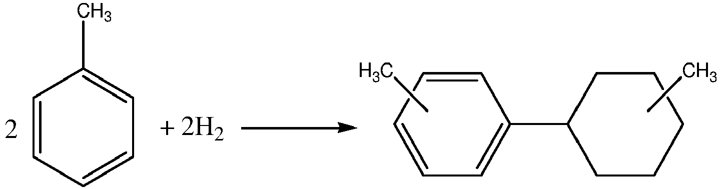

Thus, where a mixture of Cₙ - C₁₂ aromatic hydrocarbons is present in the hydroalkylation feed, a mixture of corresponding C₂ₙ - C₂₄ cycloalkylaromatic compounds will be formed in the hydroalkylation reaction, where n ranges from 6 - 12.

Various reactions also compete with the hydroalkylation of the aromatic hydrocarbon. Among the competing reactions is further hydrogenation of the above-noted cyclic olefin intermediate and/or the cycloalkylaromatic product to produce fully saturated rings, such as one or more C₆ - C₁₂ saturated cyclic hydrocarbons. Again returning to the example of toluene as the hydroalkylation feed, further hydrogenation can produce methylcyclohexane (a C₇ saturated cyclic hydrocarbon) and dimethylbicyclohexane compounds. Although these by-products can be converted back to feed (e.g., toluene) and to the product (e.g., (methylcyclohexyl)toluene and dimethylbiphenyl) via dehydrogenation, this involves an endothermic reaction requiring high temperatures (>375°C) to obtain high conversion. This not only makes the reaction costly but can also lead to further by-product formation and hence yield loss. It is therefore desirable to employ a hydroalkylation catalyst that exhibits low selectivity towards the production of fully saturated rings.

Another competing reaction is dialkylation of the desired cycloalkylaromatic compound product. Again returning to the example of toluene hydroalkylation (in which (methylcyclohexyl)toluene is produced), the (methylcyclohexyl)toluene product reacts with further methylcyclohexene to produce di(methylcyclohexyl)toluene. Again this by-product can be converted back to (methylcyclohexyl)toluene, in this case by transalkylation. However, this process requires the use of an acid catalyst at temperatures above 160°C and can lead to the production of additional by-products, such as di(methylcyclopentyl)toluenes, cyclohexylxylenes and cyclohexylbenzene. It is therefore desirable to employ a hydroalkylation catalyst that exhibits low selectivity towards di(methylcyclohexyl)toluene and other heavy by-products.

In addition to the aromatic hydrocarbon and hydrogen, a diluent, which is substantially inert under hydroalkylation conditions, may be included in the feed to the hydroalkylation reaction. In certain embodiments, the diluent is a hydrocarbon, in which the desired cycloalkylaromatic product is soluble, such as a straight chain paraffinic hydrocarbon, a branched chain paraffinic hydrocarbon, and/or a cyclic paraffinic hydrocarbon. Examples of suitable diluents are decane and cyclohexane. Although the amount of diluent is not narrowly defined, desirably the diluent is added in an amount such that the weight ratio of the diluent to the aromatic compound is at least 1:100; for example at least 1:10, but no more than 10:1, desirably no more than 4:1.

The hydroalkylation reaction can be conducted in a wide range of reactor configurations including fixed bed, slurry reactors, and/or catalytic distillation towers. In addition, the hydroalkylation reaction can be conducted in a single reaction zone or in a plurality of reaction zones, in which at least the hydrogen is introduced to the reaction in stages. Suitable reaction temperatures are between about 100°C and about 400°C, such as between about 125°C and about 250°C, while suitable reaction pressures are between about 100 and about 7,000 kPa, such as between about 500 and about 5,000 kPa. The molar ratio of hydrogen to aromatic feed is typically from about 0.15:1 to about 15:1.

The catalyst employed in the hydroalkylation reaction is a bifunctional catalyst comprising a hydrogenation component (e.g., a hydrogenation metal selected from group 10 of the Periodic Table of the Elements, with palladium being particularly advantageous) and a solid acid alkylation component, typically a molecular sieve. The catalyst may also include a binder such as clay, silica and/or metal oxides. In general, suitable hydroalkylation catalysts include those described in Paragraphs [0025] - [0029] of WIPO Publication No. 2014/159104 (published 2 October 2014, with International Filing Date of 7 March 2014).

A particularly preferred hydroalkylation catalyst, as noted therein, comprises a molecular sieve of the MCM-22 family. Molecular sieves of MCM-22 family generally have an X-ray diffraction pattern including d-spacing maxima at 12.4±0.25, 6.9±0.15, 3.57±0.07 and 3.42±0.07 Angstrom. The X-ray diffraction data used to characterize the material are obtained by standard techniques using the K-alpha doublet of copper as the incident radiation and a diffractometer equipped with a scintillation counter and associated computer as the collection system. Molecular sieves of MCM-22 family include MCM-22 (described in US 4,954,325), PSH-3 (described in US 4,439,409), SSZ-25 (described in US 4,826,667), ERB-1 (described in EP 0 293 032), ITQ-1 (described in US 6,077,498), ITQ-2 (described in WO 97/17290), MCM-36 (described in US 5,250,277), MCM-49 (described in US 5,236,575), MCM-56 (described in US 5,362,697) and mixtures thereof.

MCM-22 family molecular sieves are particularly active and stable catalysts for the hydroalkylation of toluene or xylene. In addition, catalysts containing MCM-22 family molecular sieves exhibit improved selectivity to the 3,3'-dimethyl, the 3,4'-dimethyl, the 4,3'-dimethyl and the 4,4'-dimethyl isomers in the hydroalkylation product, while at the same time reducing the formation of fully saturated and heavy by-products. For example, using an MCM-22 family molecular sieve with a toluene feed, it is found that the hydroalkylation reaction effluent may comprise:
- at least 60 wt%, such as at least 70 wt%, for example at least 80 wt% of the 3,3, 3,4, 4,3 and 4,4-isomers of (methylcyclohexyl)toluene based on the total weight of all the (methylcyclohexyl)toluene isomers;
- less than 30 wt% of methylcyclohexane and less than 5 wt% of dimethylbicyclohexane compounds; and
- less than 3 wt% of compounds containing in excess of 14 carbon atoms.

Similarly, with a xylene feed, the hydroalkylation reaction effluent may comprise less than 3 wt% of compounds containing in excess of 16 carbon atoms. Likewise, with a diethylbenzene feed, the hydroalkylation reaction effluent may comprise less than 3 wt % of compounds containing in excess of 20 carbon atoms.

By way of illustration, the 3,3, 3,4 4,3 and 4,4-isomers of (methylcyclohexyl)toluene are illustrated in formulas F1 to F4, respectively:

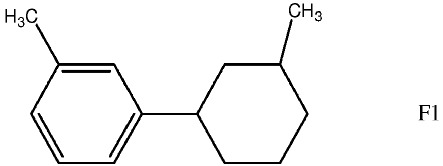

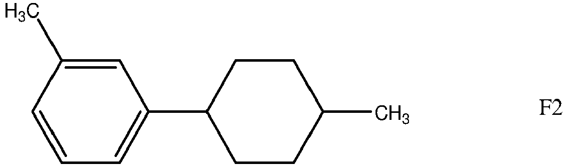

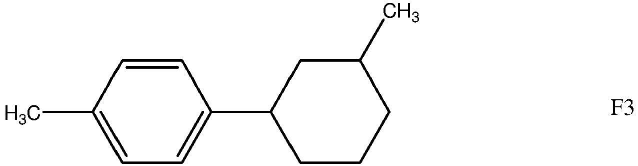

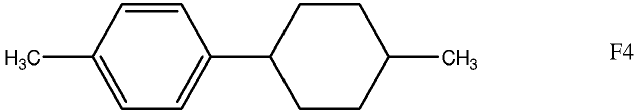

In contrast, when the methyl group is located in the 1-position (quaternary carbon) on the cyclohexyl ring, ring isomerization can occur forming (dimethylcyclopentyl)toluene and (ethylcyclopentyl)toluene which, on dehydrogenation, will generate diene by-products which are difficult to separate from the desired product and will also inhibit the subsequent oxidation reaction. In the oxidation and esterification steps, different isomers have different reactivity. Thus, para-isomers are more reactive than meta-isomers which are more reactive than ortho-isomers. Also in the dehydrogenation step, the presence of a methyl group in the 2 position on either the cyclohexyl or phenyl ring is a precursor for the formation of fluorene and methyl fluorene. Fluorene is difficult to separate from the dimethylbiphenyl product and causes problems in the oxidation step and also in the performance of plasticizers formed from the biphenyl compounds. It is therefore advantageous to minimize the formation of isomers which have a methyl group in the ortho, 2 and benzylic positions.

### Dehydrogenation of Hydroalkylation Reaction Effluent

In processes according to some embodiments in which a hydroalkylation feed comprising a Cₙ aromatic hydrocarbon is used, the major components of the hydroalkylation reaction effluent include: (i) unreacted Cₙ aromatic hydrocarbons from the hydroalkylation feed (e.g., toluene or xylene); (ii) Cₙ saturated cyclic hydrocarbon byproducts (e.g., methylcyclohexane or dimethylcyclohexane); (iii) the desired C₂ₙ cycloalkylaromatic product (e.g., (methycyclohexyl)toluene or (dimethylcyclohexyl)xylene; and (iv) some dialkylated C₃ₙ or greater hydrocarbons. The identity of the dialkylated C₃ₙ or greater hydrocarbons will depend at least in part upon the aromatic hydrocarbon(s) present in the feed. Benzene (a Cₙ aromatic hydrocarbon where n = 6), for example, will lead to production of some C₁₈ (i.e., C₃ₙ) dialkylated species (dicyclohexylbenzene), while toluene (C₇) will lead to production of some C₂₁ dialkylated species (di(methylcyclohexyl)toluene), etc. These species are labeled "dialkylated" because they have undergone the alkylation portion of the hydroalkylation reaction twice (e.g., a benzene, toluene, or the like has been alkylated with cyclic alkane twice).

As noted previously, it is also contemplated that the hydroalkylation feed may include mixtures of multiple species of Cₙ - C₁₂ aromatic hydrocarbons. In other words, the feed may further include, in addition to the Cₙ aromatic hydrocarbons, one or more Cₙ₊₁ - C₁₂ aromatic hydrocarbons. In such cases, the cycloalkylated products will further comprise C₂ₙ₊₁ - C₂₄ cycloalkylated compounds corresponding to the species in the feed. Likewise, the saturated cyclic hydrocarbon byproducts will further include Cₙ₊₁ - C₁₂ saturated cyclic hydrocarbons corresponding to the aromatic species in the feed.

It is desired to dehydrogenate both the C₂ₙ cycloalkylaromatic compounds and the Cₙ saturated cyclic hydrocarbon byproducts, such that the dehydrogenated cycloalkylaromatic compounds form the desired biphenyl compounds, and the dehydrogenated saturated cyclic hydrocarbons form additional aromatic hydrocarbon that may be recycled together with any unreacted aromatic hydrocarbons to provide additional hydroalkylation feed. Thus, the process may further comprise, after hydroalkylation, separating the dialkylated C₃ₙ or greater hydrocarbons from the hydroalkylation reaction effluent, leaving only the compounds to be dehydrogenated (cycloalkylaromatic compounds and saturated cyclic hydrocarbons) and the unreacted aromatic hydrocarbons to be recycled to the hydroalkylation reaction.

At this point, despite the desire to dehydrogenate both the cycloalkylaromatic compounds and saturated cyclic hydrocarbons, it is advantageous to perform at least one dehydrogenation of the Cₙ saturated cyclic hydrocarbons separately from, or in the substantial absence of, C₂ₙ or greater hydrocarbons, including the C₂ₙ cycloalkylaromatic compounds. Thus, processes of certain embodiments include dehydrogenating at least a portion of the Cₙ saturated cyclic hydrocarbons in the presence of less than about 5 wt%, more preferably less than about 1 wt%, even more preferably less than about 0.1 wt%, most preferably less than 0.01 wt%, of C₂ₙ or greater hydrocarbons, where n is an integer from 6 to 12. Particular embodiments include dehydrogenating at least a portion of the Cₙ cyclic hydrocarbons in the presence of less than about 5 wt%, more preferably less than about 1 wt%, even more preferably less than about 0.1 wt%, most preferably less than about 0.01 wt%, of C₂ₙ or greater (such as C₂ₙ - C₂₄) cycloalkylaromatic compounds and/or C₃ₙ or greater dialkylated hydrocarbons from the hydroalkylation reaction effluent. Conversion rate in the dehydrogenation of the saturated cyclic hydrocarbons is thereby substantially improved. Where one or more Cₙ₊₁ - C₁₂ cyclic hydrocarbons also need to be dehydrogenated, preferably at least a portion of the Cₙ₊₁ - C₁₂ cyclic hydrocarbons are also dehydrogenated in the presence of less than about 5 wt%, more preferably less than about 1 wt%, even more preferably less than about 0.1 wt%, most preferably less than about 0.01 wt%, of C₂ₙ or greater compounds. It will be appreciated that, where n=6, the portion of the Cₙ₊₁ - C₁₂ cyclic hydrocarbons to be preferably dehydrogenated separately from the C₂ₙ or greater compounds will comprise only the Cₙ₊₁ - C₁₁ cyclic hydrocarbons.

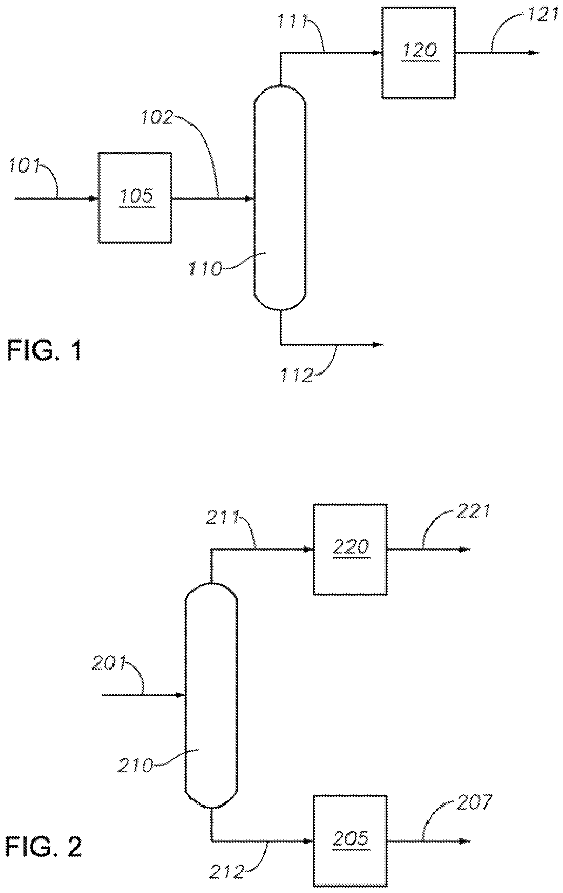

Accordingly, processes according to some embodiments include (i) dehydrogenation of at least a portion of the hydroalkylation effluent, which comprises both components (Cₙ saturated cyclic hydrocarbons and C₂ₙ cycloalkylaromatic compounds); and (ii) subsequent dehydrogenation of at least a portion of the Cₙ saturated cyclic hydrocarbons in the presence of less than about 5 wt% (more preferably less than about 1 wt%, even more preferably less than any one of about 0.1 wt% and 0.01 wt%) C₂ₙ or greater hydrocarbons (e.g., the cycloalkylaromatic compounds), where n is an integer from 6 to 12. In particular embodiments, n is 7 (e.g., for toluene feed) or 8 (e.g., for xylene feed). Such processes are illustrated in the simplified process flow diagram of Figure 1.

Alternatively, processes according to other embodiments include: (i) first separating the Cₙ saturated cyclic hydrocarbons from the C₂ₙ or greater hydrocarbons (including C₂ₙ cycloalkylaromatic compounds); and then (ii) dehydrogenating at least a portion of the Cₙ saturated cyclic hydrocarbons in the presence of less than about 5 wt% (more preferably less than about 1 wt%, even more preferably less than any one of about 0.1 wt% and 0.01 wt%) C₂ₙ or greater hydrocarbons (including the cycloalkylaromatic compounds). The C₂ₙ cycloalkylaromatic compounds may also be dehydrogenated in an additional dehydrogenation reaction. Such processes are illustrated in the simplified process flow diagram of Figure 2. As in embodiments in accordance with Figure 1, n is an integer from 6 to 12 in embodiments in accordance with Figure 2. In certain of these embodiments, n is 7 (e.g., for hydroalkylation feed including toluene as the smallest C₆ - C₁₂ aromatic hydrocarbon) or 8 (e.g., for hydroalkylation feed including xylene as the smallest C₆ - C₁₂ aromatic hydrocarbon).

Each of the processes according to Figure 1 and the processes according to Figure 2 are discussed in greater detail below.

First, processes of some embodiments in accordance with Figure 1 include: providing at least a portion of the hydroalkylation reaction effluent (shown in process stream 101) to a first dehydrogenation zone 105 to obtain a first dehydrogenation reaction effluent (shown in process stream 102). The desired reactions are: (i) dehydrogenation of the C₂ₙ - C₂₄ cycloalkylaromatic compounds to corresponding biphenyl compounds, and (ii) dehydrogenation of Cₙ - C₁₂ saturated cyclic hydrocarbons to corresponding Cₙ - C₁₂ aromatic hydrocarbons (where n is an integer between 6 and 12, such as 7 or 8 in particular embodiments). However, because the dehydrogenation of the Cₙ saturated cyclic hydrocarbons in the presence of C₂ₙ or greater hydrocarbons (e.g., the C₂ₙ cycloalkylaromatic compounds) will be incomplete, the first dehydrogenation reaction effluent in process stream 102 comprises (i) C₂ₙ biphenyl compounds; (ii) Cₙ aromatic hydrocarbons; and (iii) unreacted Cₙ saturated cyclic hydrocarbons. In some embodiments, the first dehydrogenation reaction effluent may further comprise (iv) unreacted C₂ₙ cycloalkylaromatic compounds.

The C₂ₙ and greater compounds (including the C₂ₙ biphenyl compounds, and any unreacted C₂ₙ cycloalkylaromatic compounds) are separated from the first dehydrogenation reaction effluent, e.g., via fractionation. In particular, the first dehydrogenation reaction effluent is separated into a heavy dehydrogenation effluent rich in the C₂ₙ compounds (including, but not necessarily limited to, the C₂ₙ biphenyl compounds and any unreacted C₂ₙ cycloalkylaromatic compounds) and a light dehydrogenation effluent rich in (a) the Cₙ saturated cyclic hydrocarbon and (b) the Cₙ aromatic hydrocarbon, where n is an integer from 6 to 12 (such as 7 or 8, in particular embodiments). The light dehydrogenation effluent may further comprise, where applicable, one or more Cₙ₊₁ - C₁₂ saturated cyclic hydrocarbons and/or aromatic hydrocarbons (except where n = 6, in which chase the light dehydrogenation effluent may further comprise one or more Cₙ₊₁ - C₁₁ saturated cyclic hydrocarbons and/or aromatic hydrocarbons). Likewise, the heavy dehydrogenation effluent may comprise one or more C₂ₙ₊₂ - C₂₄ cycloalkylaromatic compounds. When an effluent, product, or other like mixture in a process stream is described herein as being "rich," "rich in," or "enriched" in one or more specified species, it is meant that the wt% of each specified species in that stream is enriched relative to the feed stream prior to separation. On the other hand, when a stream is described as being "depleted" in one or more specified species, it is meant that the wt% of each specified species in that stream is reduced relative to the feed stream prior to separation. Thus, a post-separation stream is "rich in Cₙ saturated cyclic hydrocarbons and Cₙ aromatic hydrocarbons" when the wt% of each of the Cₙ saturated cyclic hydrocarbons and Cₙ aromatic hydrocarbons in that stream is enriched (greater) relative to the wt% of each species in the corresponding stream prior to separation.

For example, Figure 1 shows such separation in a distillation column 110, such that bottoms process stream 112 carries away the heavy dehydrogenation effluent, and top process stream 111 carries away the light dehydrogenation effluent.

The C₂ₙ biphenyl compounds of the heavy dehydrogenation effluent may be used in forming biphenyl ester plasticizers, as is described in greater detail below. Optionally, the heavy dehydrogenation effluent may be subjected to further separations and/or other treatment to obtain a product comprising at least 90 wt%, preferably at least 95%, more preferably at least 99 wt% biphenyl compounds. Optionally, at least a portion of the heavy dehydrogenation effluent may be recycled to the first dehydrogenation zone (e.g., to dehydrogenate unreacted cycloalkylaromatic compounds, if any, present in the heavy dehydrogenation effluent).

In particular embodiments, the light dehydrogenation effluent comprises less than about 5 wt%, preferably less than about 1 wt%, more preferably less than about 0.5 wt%, even more preferably less than about 0.1 wt%, and most preferably less than about 0.01 wt% of C₂ₙ or greater hydrocarbons (including, e.g., C₂ₙ cycloalkylaromatic compounds and/or C₂ₙ biphenyl compounds). That is to say, preferably, the separation of the first dehydrogenation effluent is carried out such that C₂ₙ and greater hydrocarbons are recovered in the bottoms fraction, and substantially not present in the light fraction. The exact conditions of the separation will depend at least in part upon the species present in the first dehydrogenation effluent. For instance, where the Cₙ aromatic hydrocarbons fed to the initial hydroalkylation comprise toluene (C₇), it is expected that C₁₄ cycloalkylaromatic compounds (e.g., (methylcyclohexyl)toluene) and C₁₄ biphenyl compounds (e.g., dimethylbiphenyl) will be formed from hydroalkylation and dehydrogenation, respectively, while unreacted toluene and dehydrogenated unreacted toluene (i.e., methylcyclohexane, a C₇ saturated cyclic hydrocarbon) will also be present in the first dehydrogenation effluent. Such separation would therefore be run so as to remove a heavy dehydrogenation effluent of C₁₄ (and greater, as applicable) compounds, and a light dehydrogenation effluent of the C₇ compounds (which may further comprise any C₈ - C₁₂ compounds, such as any unreacted C₈ - C₁₂ aromatic hydrocarbons and/or saturated cyclic hydrocarbons, if present). An ordinarily skilled artisan will readily recognize suitable separation conditions, such as suitable distillation pressure and temperature, to separate the two sets of species Cₙ and C₂ₙ, given their amply different volatilities.

The light dehydrogenation effluent is subjected to a second dehydrogenation reaction in a second dehydrogenation zone 120 separate from the first dehydrogenation zone 105 to provide a second dehydrogenation effluent leaving in process stream 121. In the second dehydrogenation reaction, at least a portion of the Cₙ saturated cyclic hydrocarbons are dehydrogenated to corresponding Cₙ aromatic hydrocarbons (e.g., methylcyclohexane would be dehydrogenated to toluene). In some embodiments, at least about 90, preferably 95, more preferably 99, most preferably 99.9 wt% of the Cₙ saturated cyclic hydrocarbons of the light dehydrogenation effluent are dehydrogenated in a single pass in the second dehydrogenation zone 120. In some embodiments, then, the resulting second dehydrogenation effluent (shown in process stream 121) may comprise less than 1 wt% (e.g., less than any one of 0.5 wt%, 0.1 wt%, and 0.01 wt%) Cₙ saturated cyclic hydrocarbons. Further, in certain embodiments, the second dehydrogenation effluent comprises greater than about 80 wt%, preferably greater than about 90 wt%, more preferably greater than 95 wt%, most preferably greater than about 99 wt% Cₙ aromatic hydrocarbons. The second dehydrogenation product may optionally be recycled to provide additional aromatic hydrocarbons to the hydroalkylation feed (not shown in Fig. 1).

Optionally, the second dehydrogenation product may be purified prior to being combined with the hydroalkylation feed. Any suitable separation technique may be used for such purification, such as fractionation, filtration, adsorption, absorption, and the like.

However, in some particularly advantageous embodiments, the dehydrogenation of Cₙ saturated cyclic hydrocarbons separate from the C₂ₙ species, as provided herein, may facilitate conversions high enough so that distillation (or other like purification) after such separate dehydrogenation may not be necessary before recycling such dehydrogenation product back as hydroalkylation feed. For instance, in the case of MCH, the MCH concentration may be low enough coming out of such separate dehydrogenation such that MCH will not build up, interfere with, or substantially increase the recycle stream size for the hydroalkylation reactor. This can advantageously avoid the need to separate unreacted saturated cyclic hydrocarbons (e.g., MCH) from the recycled aromatic feed (e.g., toluene).

In further embodiments, purification (e.g., fractionation) of the second dehydrogenation product may still take place, but such purification may be operated at less severe conditions than would be required to separate a Cₙ saturated cyclic hydrocarbon from its corresponding Cₙ aromatic hydrocarbon (e.g., separating MCH from toluene, using the same example).

As noted, Figure 2 provides a simplified illustration of processes according to other embodiments. In particular, such processes include separating the hydroalkylation reaction effluent (shown in process stream 201) into (i) a heavy hydroalkylation effluent rich in C₂ₙ and greater hydrocarbons (including the C₂ₙ cycloalkylaromatic compounds from the hydroalkylation reaction effluent), and (ii) a light hydroalkylation effluent rich in the Cₙ saturated cyclic hydrocarbons from the hydroalkylation reaction effluent (where n is an integer from 6 to 12, such as 7 or 8, in particular embodiments). The light hydroalkylation effluent may, where applicable, further comprise one or more Cₙ₊₁ - C₁₂ saturated cyclic hydrocarbons (or, where n = 6, it may further comprise one or more Cₙ₊₁ - C₁₁ saturated cyclic hydrocarbons). Similarly, the heavy hydroalkylation effluent may further comprise, where applicable, one or more C₂ₙ₊₂ - C₂₄ cycloalkylaromatic compounds.

The separation may be accomplished by any suitable means, such as fractionation. For example, in Figure 2, the hydroalkylation effluent in process stream 201 is provided to a distillation column 210, with bottoms process stream 212 carrying away the heavy hydroalkylation effluent and top process stream 211 carrying away the light hydroalkylation effluent. In some embodiments, the light hydroalkylation effluent comprises less than about 5 wt%, preferably less than about 1 wt%, more preferably less than about 0.5 wt%, even more preferably less than about 0.1 wt%, and most preferably less than about 0.01 wt% of C₂ₙ or greater hydrocarbons (e.g., C₂ₙ cycloalkylaromatic compounds).

The process further comprises providing at least a portion of the heavy hydroalkylation effluent to a first dehydrogenation zone, and providing at least a portion of the light hydroalkylation effluent to a second dehydrogenation zone separate from the first. As shown in Figure 2, the heavy hydroalkylation effluent is provided to a first dehydrogenation zone 205 via bottoms process stream 212, and the light hydroalkylation effluent is provided to a second dehydrogenation zone 220 via top process stream 211.

At least a portion of the heavy hydroalkylation reaction effluent is dehydrogenated in the first dehydrogenation reaction zone 205, providing a heavy dehydrogenation reaction product comprising a mixture of C₁₂ - C₂₀ biphenyl compounds, shown in process stream 207 in Figure 2. The biphenyl compounds may be useful in forming biphenyl ester plasticizers, as described below. The heavy dehydrogenation reaction product may further comprise unreacted C₂ₙ cycloalkylaromatic compounds. Accordingly, at least a portion of the heavy dehydrogenation reaction product may optionally be recycled to the first dehydrogenation zone to provide additional cycloalkylaromatic compound feed for the first dehydrogenation reaction (not shown in Figure 2).

Similarly, at least a portion of the light hydroalkylation reaction effluent is dehydrogenated in the second dehydrogenation reaction zone 220, providing a light dehydrogenation reaction product comprising a recovered Cₙ aromatic hydrocarbon (e.g., toluene and/or xylene), shown in process stream 221 in Figure 2. The light dehydrogenation reaction product may further comprise a recovered Cₙ₊₁ - C₁₂ recovered hydrocarbon (or, where n = 6, a recovered Cₙ₊₁ - C₁₁ recovered hydrocarbon). At least a portion of the recovered aromatic hydrocarbon(s) may be recycled to provide additional hydroalkylation feed (not shown in Figure 2). In the dehydrogenation of the light hydroalkylation reaction effluent in the second dehydrogenation zone, at least about 90, preferably 95, more preferably 99, most preferably 99.9, wt% of the Cₙ saturated cyclic hydrocarbons of the light hydroalkylation reaction effluent are dehydrogenated. Accordingly, the light dehydrogenation reaction product in process stream 221 may comprise less than 1 wt%, preferably less than 0.5 wt%, more preferably less than 0.1 wt% Cₙ saturated cyclic hydrocarbons.

Further, in some embodiments, the light dehydrogenation reaction product may be further purified in any manner discussed above respecting the second dehydrogenation product of processes in accordance with Figure 1. Alternatively, also as discussed with respect to processes in accordance with Figure 1, it may advantageously not be necessary to provide for any purification of the light dehydrogenation reaction product; or, similarly, less severe purification conditions may be used. That is, as with processes in accordance with Figure 1, any purification of the light dehydrogenation reaction product may be operated so as to remove higher or lower hydrocarbon species from the aromatic hydrocarbon that is desired to be recycled as hydroalkylation feed, but such purification need not be run at the (more severe) conditions necessary to separate a Cₙ saturated cyclic hydrocarbon from its corresponding Cₙ aromatic hydrocarbon.

In some embodiments according to any of the above-described processes (e.g., in accordance with either Figure 1 or Figure 2), as noted, the hydroalkylation reaction effluent comprises dialkylated C₃ₙ or greater hydrocarbons (where n, again, is an integer from 6 to 12). As also previously noted, such dialkylated C₃ₙ or greater hydrocarbons may be removed from the hydroalkylation reaction effluent prior to either or both dehydrogenation reactions. For example, in embodiments according to Figure 1, C₃ₙ or greater hydrocarbons may be removed from the hydroalkylation effluent in process stream 101 via an additional distillation (or other suitable separation) step prior to the hydroalkylation effluent being provided to the first dehydrogenation zone 105. In embodiments according to Figure 2, dialkylated C₃ₙ or greater hydrocarbons may be removed from the hydroalkylation effluent in a similar manner (e.g., additional distillation or other separation carried out on the hydroalkylation effluent in process stream 201) prior to the hydroalkylation reaction effluent being separated (e.g., at distillation column 210) into the light hydroalkylation effluent in stream 211 and the heavy hydroalkylation effluent in stream 212. Alternatively, the dialkylated C₃ₙ or greater hydrocarbons may be separated into the heavy hydroalkylation effluent in process stream 212 along with the C₂ₙ or greater hydrocarbons (e.g., the C₂ₙ cycloalkylaromatic compounds). An additional separation may therefore be employed in process stream 212 prior to the first dehydrogenation zone 205 to separate the dialkylated C₃ₙ or greater compounds from the C₂ₙ cycloalkylaromatic compounds in the heavy hydroalkylation effluent.

Each dehydrogenation zone in the processes of various embodiments (e.g., first and second dehydrogenation zones 105 and 120 in processes according to Figure 1, and/or first and second dehydrogenation zones 205 and 220 in processes according to Figure 2) may comprise one or more dehydrogenation reactors operating in series, in parallel, or in any combination thereof. Each dehydrogenation reaction is conducted at a temperature from about 200°C to about 600°C and a pressure from about 100 kPa to about 3550 kPa (atmospheric to about 500 psig) in the presence of dehydrogenation catalyst. In particular embodiments, dehydrogenation may be conducted at a temperature of from about 250°C to about 500°C, preferably from about 300°C to about 450°C, or from about 375°C to about 450°C. Hydrogen may be co-fed with the hydrocarbons to the dehydrogenation reaction (e.g., for catalyst stability purposes as described in co-pending U.S. Provisional Application No. 62/068,144). Where hydrogen is co-fed, the feed ratio (in terms of moles H₂ to moles hydrocarbons) may be within the range of about 0.5 to about 4.0, such as about 1.0 to about 3.0. Each dehydrogenation reactor may independently be any reactor suitable for dehydrogenation at such conditions (e.g., adiabatic fixed-beds in series, isothermal fixed-beds, etc.). Each dehydrogenation may be run at approximately the same conditions, or at different conditions. For instance, in some embodiments, dehydrogenation of the lighter cut (e.g., of the light hydroalkylation reaction effluent in embodiments in accordance with Figure 2, or the light dehydrogenation effluent of embodiments in accordance with Figure 1) may be run at a slightly higher temperature than dehydrogenation of a heavier cut.

The same or different dehydrogenation catalysts may be used in the dehydrogenation reactions described above. In general, suitable dehydrogenation catalysts include any dehydrogenation catalyst in accordance with those described in paragraphs [0055] - [0072] of US 2014/0275607. In some embodiments, as discussed therein, the dehydrogenation catalyst comprises one or more elements or compounds thereof selected from group 10 of the Periodic Table of Elements, for example platinum, on a refractory support. In one embodiment, the group 10 element is present in amount from 0.1 to 5 wt% of the catalyst. In some cases, the dehydrogenation catalyst may also include tin or a tin compound to improve the selectivity to the desired methyl-substituted biphenyl product. In one embodiment, the tin is present in amount from 0.05 to 2.5 wt% of the catalyst. In other embodiments, the tin may preferably be present in an amount from about 0.05 to about 0.30 wt%, more preferably from about 0.05 to about 0.15 wt%. The support employed in the dehydrogenation catalyst is refractory in the sense that it is capable of withstanding the conditions employed in the dehydrogenation reaction without physical or chemical changes. Non-limiting examples of suitable refractory support materials include: alumina, silica, silica-alumina, titania, calcium oxide, strontium oxide, barium oxide, magnesium oxide, carbon, zirconia, diatomaceous earth, lanthanide oxides including cerium oxide, lanthanum oxide, neodynium oxide, yttrium oxide and praesodynium oxide, oxides of chromium, thorium, uranium, niobium and tantalum, tin oxide, zinc oxide, and aluminum phosphate.

Further, as also discussed in US 2014/0275607, where the dehydrogenation catalyst contains tin, the catalyst may be prepared by impregnating the support with an aqueous solution of a suitable tin compound, such as tin chloride, and/or tin tartrate. The impregnated support containing Sn is then dried in air, such as at about 110°C to about 130°C (e.g., about 120°C) for 4 hours, and then calcined, such as at about 350 to about 600°C in air for 3 hours, to convert the tin to an oxide form. Afterwards, Pt is added to Sn-containing support by impregnation with an aqueous solution of a suitable platinum compound, such as (NH₃)₄Pt(NO₃)₂. The sample containing Sn and Pt is dried in air, such as at 120°C for 4 hours, and then calcined, such as at a temperature between about 330°C to about 370°C (preferably about 350°C to about 360°C) in air for 3 hours.

In addition or instead, the dehydrogenation catalyst may be according to those described in Paragraphs [0020] to [0030] of US 2014/0323782.

Processes utilizing multiple dehydrogenation zones according to some embodiments may use the same dehydrogenation catalyst in each of the multiple dehydrogenation zones. In some such embodiments where Cₙ saturated cyclic hydrocarbons are dehydrogenated at least in part separately from C₂ₙ and greater hydrocarbons, less overall dehydrogenation catalyst may be used as compared to a process in which all dehydrogenation of Cₙ saturated cyclic hydrocarbons takes place in the presence of C₂ₙ and greater hydrocarbons.

### Production of Biphenyl Esters

Methyl-substituted biphenyl compounds (e.g., dimethylbiphenyl) produced by the processes of some embodiments can readily be converted to ester plasticizers by a process comprising oxidation to produce the corresponding carboxylic acids followed by esterification with an alcohol.

The oxidation can be performed by any process known in the art, such as by reacting the methyl-substituted biphenyl compounds with an oxidant, such as oxygen, ozone or air, or any other oxygen source, such as hydrogen peroxide, in the presence of a catalyst at temperatures from 30°C to 300°C, such as from 60°C to 200°C. Suitable catalysts comprise Co or Mn or a combination of both metals. Alternatively or in addition, the oxidation may take place in the presence of Br as an initiator.

The resulting carboxylic acids can then be esterified to produce biphenyl ester plasticizers by reaction with one or more C₄ to C₁₄ alcohols. Suitable esterification conditions are well-known in the art and include, but are not limited to, temperatures of 0-300°C and the presence or absence of homogeneous or heterogeneous esterification catalysts, such as Lewis or Bronsted acid catalysts. Suitable alcohols include "oxo-alcohols," by which is meant an organic alcohol, or mixture of organic alcohols, which is prepared by hydroformylating an olefin, followed by hydrogenation to form the alcohols. Typically, the olefin is formed by light olefin oligomerization over heterogeneous acid catalysts, which olefins are readily available from refinery processing operations. The reaction results in mixtures of longer-chain, branched olefins, which subsequently form longer chain, branched alcohols, as described in US 6,274,756. Another source of olefins used in the OXO process are through the oligomerization of ethylene, producing mixtures of predominately straight chain alcohols with lesser amounts of lightly branched alcohols.

The biphenyl ester plasticizers of the present application find use in a number of different polymers, such as vinyl chloride resins, polyesters, polyurethanes, ethylene-vinyl acetate copolymers, rubbers, poly(meth)acrylics and mixtures thereof.

The invention will now be more particularly described with reference to the following non-limiting Examples.

### EXAMPLES

### Example 1: General Procedure for Toluene Hydroalkylation

A palladium MCM-49 hydroalkylation catalyst was loaded into a hydroalkylation reactor. The reactor comprised a stainless steel tube having an outside diameter of: 3/8 inch (0.95 cm), a length of 20.5 inch (52 cm) and a wall thickness of 0.35 inch (0.9 cm). A piece of stainless steel tubing having a length of 8¾ inch (22 cm) and an outside diameter of: 3/8 inch (0.95 cm) and a similar length of ¼ inch (0.6 cm) tubing of were used in the bottom of the reactor (one inside of the other) as a spacer to position and support the catalyst in the isothermal zone of the furnace. A ¼ inch (0.6 cm) plug of glass wool was placed on top of the spacer to keep the catalyst in place. A 1/8 inch (0.3 cm) stainless steel thermo-well was placed in the catalyst bed to monitor temperature throughout the catalyst bed using a movable thermocouple.

The catalyst was sized to 20/40 sieve mesh or cut to 1:1 length to diameter ratio, dispersed with quartz chips (20/40 mesh) then loaded into the reactor from the top to a volume of 5.5 cc. The catalyst bed typically was 15 cm. in length. The remaining void space at the top of the reactor was filled with quartz chips, with a ¼ plug of glass wool placed on top of the catalyst bed being used to separate quartz chips from the catalyst. The reactor was installed in a furnace with the catalyst bed in the middle of the furnace at a pre-marked isothermal zone. The reactor was then pressure and leak tested typically at 300 psig (2170 kPa).

The catalyst was pre-conditioned in situ by heating to 25°C to 240°C with H₂ flow at 100 cc/min and holding for 12 hours. A 500 cc ISCO syringe pump was used to introduce a chemical grade toluene feed to the reactor. Commercially available chemical grade Toluene and hydrogen feed (2:1 ratio moles H₂ to moles toluene) was pumped through a vaporizer before flowing through heated lines to the reactor. A Brooks mass flow controller was used to set the hydrogen flow rate. A Grove "Mity Mite" back pressure controller was used to control the reactor pressure typically at 150 psig (1135 kPa). GC analyses were taken to verify feed composition. The feed was then pumped through the catalyst bed held at the reaction temperature of 120°C to 180°C at a WHSV of 2 and a pressure of 150 psig (-1135.5 kPa). Non-condensable gas products were routed to an online HP 5890 GC. The liquid products exiting the reactor flowed through heated lines routed to two collection pots in series, the first pot being heated to 60°C and the second pot cooled with chilled coolant to about 10°C. Material balances were taken at 12 to 24 hour intervals. A Hewlett Packard 6890 gas chromatograph with FID detector was used for analysis.

The liquid products of the hydroalkylation reaction were found to include about 25 wt% (methylcyclohexyl)toluene (MCHT); about 8 wt% methylcyclohexane (MCH); about 66 wt% unreacted feed; and about 1 wt% byproducts.

### Example 2: Dehydrogenation of Hydroalkylation Reaction Effluent

Liquid hydroalkylation products were used as feed in a dehydrogenation reaction. The liquid hydrocarbon feed to dehydrogenation according to Example 2 comprised about 25 wt% (methylcyclohexyl)toluene (MCHT); about 8 wt% methylcyclohexane (MCH); about 66 wt% unreacted feed; and about 1 wt% byproducts. Three runs of the dehydrogenation reaction were carried out at different weight hourly space velocities (WHSVs) - in particular, WHSVs of 2, 4, and 8, corresponding to runs 2-1, 2-2, and 2-3, as shown in Table 1 (as shown in Table 1, WHSV was varied by varying the amount of catalyst loaded and maintaining the same feed rate).

**Table 1. Dehydrogenations of Hydroalkylation Reaction Effluent**

| **Run** | **Amount of Catalyst (g)** | **WHSV** | **MCH Conversion** | **MCHT conversion** |
|---|---|---|---|---|
| 2-1 | 1 | 2 | 80.5% | 83.5% |
| 2-2 | 0.5 | 4 | 60.8% | 81.5% |
| 2-3 | 0.25 | 8 | 46.9% | 76.5% |

The dehydrogenation was carried out at 430°C and 790 kPa (100 psig) in a dehydrogenation reaction system comprising 8 parallel reactors. An ISCO syringe pump was used to introduce the feed to the reactors. The liquid hydrocarbon feed was pumped through a vaporizer before being mixed in-line with H₂ at a 2:1 molar ratio of H₂ to liquid feed, then delivered to each reactor.

The 8 parallel reactors were placed in a fluidized heated sand bath to control isothermal reaction temperature. Each reactor was a U-shaped stainless steel reactor of 5mm inner diameter. Half of the U-shaped downflow path (39 cm long) was used as a preheating zone filled with quartz chips (20/40 mesh). Dehydrogenation catalyst was loaded into the upflow portion of the back-end of the U-shaped reactor, with quartz wool plugs at the top and bottom of the catalyst bed to keep it in place. Catalyst quantities ranged from about 0.25 - 1 g, depending on WHSV (as shown in Table 1).

The dehydrogenation catalyst (1 wt% platinum and 0.15 wt% tin deposited on an SiO₂ support) was prepared as follows: a 1/20" quadrulobe silica extrudate was initially impregnated with an aqueous solution of tin chloride and then dried in air at 121°C. The resultant tin-containing extrudates were then impregnated with an aqueous solution of tetraammine Platinum nitrate and again dried in air at 121°C. Each of the resultant catalyst products was then calcined in air at 350°C for 3 hours before being used in the dehydrogenation reaction.

Catalyst in all reactors was pre-conditioned in situ, starting with N₂ flow to dry the catalyst (at 50 cc/min total for all 8 reactor channels) at atmospheric pressure and 25°C. The temperature was then ramped to 80°C at a rate of 25°C/hour and held for 3 hours. The temperature was then ramped to 120°C at a rate of 25°C/hour and held at that temperature for an additional 3 hours. After the drying step, the catalysts in each reactor were reduced in H₂ at a total flow rate (combined for all reactors) of 111.2 sccm. The pressure was then increased to the reaction pressure of 790 kPa (100 psig) and held for 20 min. The temperature was then ramped to 450°C at a rate of 25°C/hour and held at this temperature for 2 hours. Finally, the temperature was reduced to the desired isothermal reaction temperature.

The products exiting the reactor system were condensed and collected in intervals (approximately one sample per day per reactor) and analyzed by GC in the same manner as indicated in Example 1.

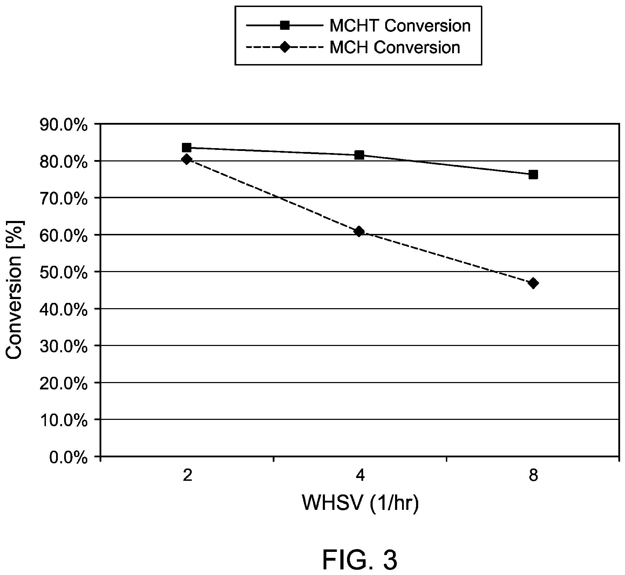

Table 1 and Figure 3 illustrate the conversion of each of MCHT (a C₁₄ cycloalkylaromatic compound) and MCH (a C₇ saturated cyclic hydrocarbon) in the dehydrogenation of Example 2. As seen in Figure 3, conversion of MCH in the presence of MCHT is at best around 80% at lowest WHSV. Furthermore, with increasing WHSV, conversion of MCH drops off (more so than the decrease in MCHT conversion), from about 80% (at 2 WHSV) to less than 50% (at 8 WHSV), therefore indicating a substantial tradeoff between process throughput and efficiency when MCH is dehydrogenated in the presence of MCHT.

### Example 3: Dehydrogenation After Separating C₂₁ and Greater Hydrocarbons

The hydroalkylation effluent from Example 1 was fractionated via batch distillation into (i) a heavy fraction comprising C₂₁ and heavier byproducts and (ii) a light fraction comprising 7 wt% MCH and 16.5 wt% MCHT, 75.7 wt% toluene, and about 0.8 wt% other hydroalkylation byproducts and impurities (including, but not limited to, cyclohexane, dimethylbicyclohexane, xylenes, and benzene).

The light fraction was provided to a dehydrogenation reactor system using the same varying feed rates (2, 4, and 8 WHSV) described in Example 2. Thus, the dehydrogenation of this example also involved dehydrogenation of both MCH and MCHT together; however, unlike the dehydrogenation of Example 2, C₂₁ and greater hydrocarbons were not present in substantial amounts. An ISCO syringe pump was used to introduce feed to the reactor system. The feed was pumped through a vaporizer before being mixed inline with H₂ at a 2:1 ratio (moles H₂ to moles liquid feed), then delivered to the reactor system. The dehydrogenation reactor system was similar to that described in Example 2, except that the 8 parallel reactors in these experiments were quartz tubes of 9mm inner diameter, and heated by furnace instead of sand bath.

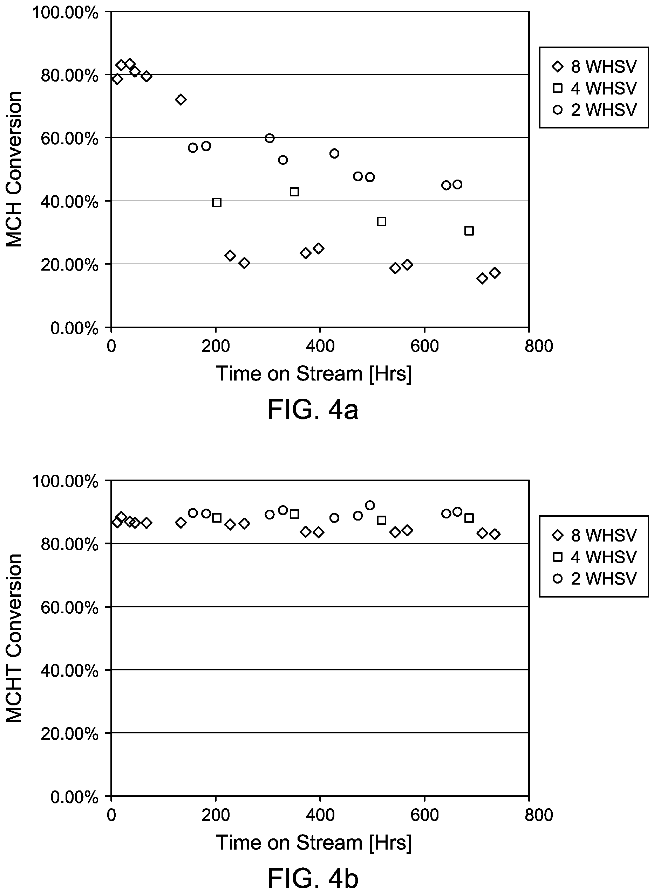

As shown in Figures 4a and 4b, even in the substantial absence of C₂₁ and greater hydrocarbons, the dehydrogenation of MCH suffers from the presence of MCHT, both at higher rates (WHSV), and at greater time on stream. As shown in Figure 4a, at 8 WHSV, MCH conversion starts at around 80%, but drops off to 70% after just over 100 hours on stream, and then to approximately 20% after 200 hours on stream. Similarly, at 4 WHSV, MCH conversion quickly drops from 80% initially to about 40% after 200 hours on stream, and from there declines to 20% after 500 hours on stream. At 2 WHSV, MCH conversion declines more steadily, reaching 60% after 200 hours on stream, and just over 40% after 600 hours on stream. By contrast, as shown in Figure 4b, MCHT conversion holds steady at over 80% at each of 2, 4, and 8 WHSV.

### Example 4: Dehydrogenation of MCH in the Absence of C₁₂ or Greater Hydrocarbons

Nearly pure MCH (>99.5% purity) was dehydrogenated over the same dehydrogenation catalyst packed in a 3/8 inch (0.95 cm) outer diameter x 16.5 inch (41.91 cm) length stainless steel reactor. The catalyst bed was centered in the isothermal zone of the reactor, with quartz wool and additional quartz chips packed into the reactor to hold the catalyst bed in place. The reactor was first purged with hydrogen and pressurized to 790 kPa (100 psig), then heated at 5°C/minute to 425°C with hydrogen flow through a first ISCO pump at 100 cc/min. Reactor temperature was maintained at 425°C for about two hours, then MCH was fed to the reactor at 2 WHSV through a second 100cc ISCO pump, and H₂ flow was reduced to 30 cc/min. Reaction temperature was maintained at 425°C, lower than the 450°C of dehydrogenation with MCH and MCHT together, as thermodynamic calculations indicated that temperatures as low as 400°C would theoretically provide 100% conversion.

Liquid product was collected in cold traps for analysis. Material balances were taken over 16 to 24 hours. A sample from each material balance was taken and analyzed on a HP 7890 GC with a Beta Dex-120 column. The Beta Dex-120 column dimensions were 60 m length x 0.25 mm outer diameter x 0.25 micron film thickness.

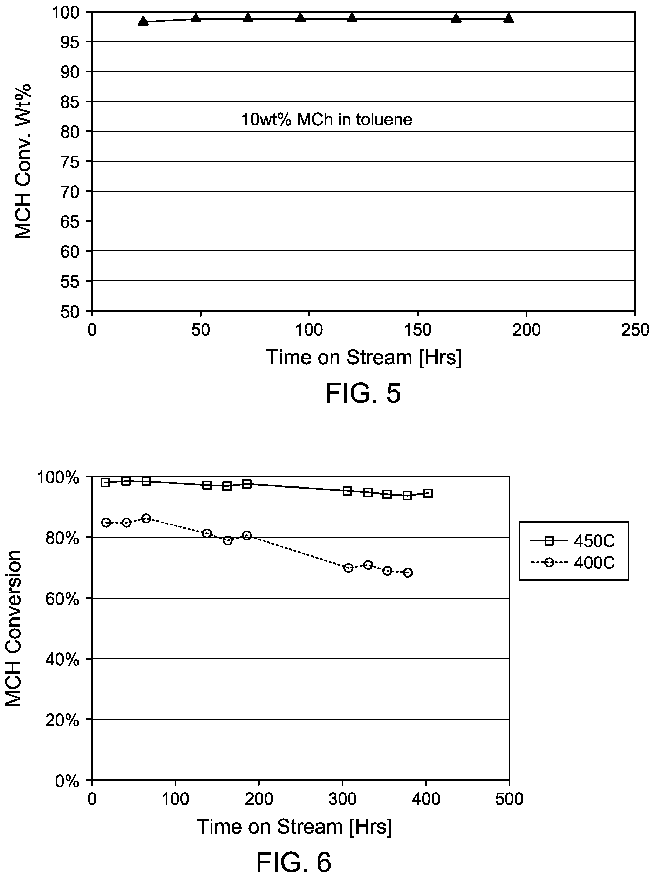

As shown in Figure 5, MCH conversion was maintained at nearly 100% for the entire time on stream (almost 200 hours), indicating significantly better conversion in the absence of heavier hydrocarbons such as the C₁₄ MCTH.

### Example 5: Dehydrogenation of Feed Comprising MCH and Toluene

Hydroalkylation effluent was dehydrogenated in the dehydrogenation reactor described in Example 3. The dehydrogenation product was distilled via batch distillation such that all heavier components, including the C₁₄ MCHT and DMBP, were under 0.1 wt% in the overhead product of the distillation. This overhead product, comprising C₇ hydrocarbons and toluene (7.8 wt% MCH, 91.9 wt% toluene, ∼ 0.3 wt% other species C₁₁ and smaller, ∼ 0.1 wt% species over C₁₁), was dehydrogenated in the same parallel reactor unit, and over a dehydrogenation catalyst, as described in Example 3. The light stream and H₂ were fed at a 1:4 ratio at 2 WHSV into the reactor at 790 kPa (100 psig). As shown in Figure 6, two different reaction temperatures were studied (400°C and 450°C). At 400°C, MCH conversion remained fairly stable, starting at around 80% and declining only slightly to about 70% after 300 hours on stream. At 450°C, MCH conversion remained steady at nearly 100% over the entire 400 hours on stream. Thus, even with toluene present, significantly higher conversion rates of MCH may be achieved by dehydrogenating MCH separately from heavier hydrocarbons.

As is apparent from the foregoing general description and the specific embodiments, while forms of the invention have been illustrated and described, various modifications can be made. Accordingly, it is not intended that the invention be limited thereby. Likewise, the term "comprising" is considered synonymous with the term "including" to require the listed components without excluding the presence of any other additional components. Likewise whenever a composition, an element or a group of elements is preceded with the transitional phrase "comprising", it is understood that we also contemplate the same composition or group of elements further narrowed with more restrictive transitional phrases such as "consisting essentially of," "consisting of', "selected from the group of consisting of," or "is" preceding the recitation of the composition, element, or elements and vice versa.

## Claims

1. A process for producing biphenyl compounds, the process comprising:
(a) contacting a hydroalkylation feed comprising Cₙ aromatic hydrocarbons with hydrogen in the presence of a hydroalkylation catalyst under conditions effective to produce a hydroalkylation reaction effluent comprising (i) C₂ₙ cycloalkylaromatic compounds and (ii) Cₙ saturated cyclic hydrocarbons, wherein n is an integer from 6 to 12;
(b) providing at least a portion of the hydroalkylation reaction effluent to a first dehydrogenation zone, therein dehydrogenating at least a portion of the C₂ₙ cycloalkylaromatic compounds and at least a portion of the Cₙ saturated cyclic hydrocarbons in the presence of a first dehydrogenation catalyst under conditions effective to produce a first dehydrogenation reaction product comprising (i) a mixture of C₂ₙ biphenyl compounds; (ii) recovered Cₙ aromatic hydrocarbons; and (iii) unreacted Cₙ saturated cyclic hydrocarbons;
(c) separating the first dehydrogenation reaction product into (i) a heavy dehydrogenation stream rich in the C₂ₙ biphenyl compounds, and (ii) a light dehydrogenation stream rich in the recovered Cₙ aromatic hydrocarbons and the unreacted Cₙ saturated cyclic hydrocarbons;
(d) providing at least a portion of the light dehydrogenation stream to a second dehydrogenation zone separate from the first dehydrogenation zone; and
(e) in the second dehydrogenation zone, dehydrogenating at least a portion of the unreacted Cₙ saturated cyclic hydrocarbons in the presence of a second dehydrogenation catalyst under conditions effective to produce a second dehydrogenation reaction product comprising additional recovered Cₙ aromatic hydrocarbons.

2. The process of claim 1, further comprising
(f) recycling at least a portion of the second dehydrogenation reaction product such that it forms at least a part of the hydroalkylation feed for the hydroalkylation step (a).

3. The process of any one of claims 1-2, wherein:
in step (a),
the Cₙ aromatic hydrocarbons are selected from the group consisting of benzene, toluene, ethylbenzene, xylene, and diethylbenzene;
the C₂ₙ cycloalkylaromatic compounds are selected from the group consisting of cyclohexylbenzene, (methylcyclohexyl)toluene, (ethylcyclohexyl) ethylbenzene, (dimethylcyclohexyl)xylene, and (diethylcyclohexyl) diethylbenzene; and
the Cₙ saturated cyclic hydrocarbons are selected from the group consisting of cyclohexane, methylcyclohexane, dimethylcyclohexane, ethylcyclohexane, and diethylcyclohexane;
in step (b),
the C₂ₙ biphenyl compounds are each selected from the group consisting of biphenyl, dimethylbiphenyl, diethylbiphenyl, tetramethylbiphenyl, and tetraethylbiphenyl;
the recovered Cₙ aromatic hydrocarbons comprise the same compound or compounds as the Cₙ aromatic hydrocarbons; and
the unreacted Cₙ saturated cyclic hydrocarbons comprise the same compound or compounds as the Cₙ saturated cyclic hydrocarbons; and
in step (e),
the additional recovered Cₙ aromatic hydrocarbons comprise the same compound or compounds as the Cₙ aromatic hydrocarbons.

4. The process of claim 3, wherein:
in step (a), the Cₙ aromatic hydrocarbons are toluene, the C₂ₙ cycloalkylaromatic compounds are (methylcyclohexyl)toluene, and the Cₙ saturated cyclic hydrocarbons are methylcyclohexane;
in step (b), the C₂ₙ biphenyl compounds are dimethylbiphenyl, the recovered Cₙ aromatic hydrocarbons are toluene, and the unreacted Cₙ saturated cyclic hydrocarbons are methylcyclohexane; and
in step (e), the additional recovered Cₙ aromatic hydrocarbons are toluene.

5. The process of claim 3 or claim 4, further comprising:
(g) contacting at least a portion of the heavy dehydrogenation stream obtained in step (c) with an oxidant under conditions effective to convert at least part of the C₂ₙ biphenyl compounds to biphenyl carboxylic acids; and
(h) reacting the biphenyl carboxylic acids with one or more C₁ - C₁₄ alcohols under conditions effective to produce biphenyl esters.

6. The process of any one of claims 1-2, wherein n is an integer from 7 to 11, and further wherein:
(I) the hydroalkylation feed in step (a) further comprises one or more Cₙ₊₁ - C₁₂ aromatic hydrocarbons;
(II) the hydroalkylation reaction effluent in step (a) further comprises (iii) one or more C₂ₙ₊₂ - C₂₄ cycloalkylaromatic compounds and (iv) one or more Cₙ₊₁ - C₁₂ saturated cyclic hydrocarbons;
(III) at least a portion of the one or more C₂ₙ₊₂ - C₂₄ cycloalkylaromatic compounds and at least a portion of the one or more Cₙ₊₁ - C₁₂ saturated cyclic hydrocarbons are dehydrogenated in the first dehydrogenation zone in step (b) along with the portion of the C₂ₙ cycloalkylaromatic compounds and the portion of the Cₙ saturated cyclic hydrocarbons, such that the first dehydrogenation reaction product in step (b) further comprises (iv) a mixture of C₂ₙ₊₂ - C₂₄ biphenyl compounds; (v) one or more recovered Cₙ₊₁ - C₁₂ aromatic hydrocarbons; and (vi) unreacted Cₙ₊₁ - C₁₂ saturated cyclic hydrocarbons; and
(IV) the light dehydrogenation stream in step (c) further comprises at least a portion of the one or more recovered Cₙ₊₁ - C₁₂ aromatic hydrocarbons and at least a portion of the unreacted Cₙ₊₁ - C₁₂ saturated cyclic hydrocarbons.

7. The process of any one of claims 1-2, wherein n is an integer from 6 to 10, and further wherein:
(I) the hydroalkylation feed in step (a) further comprises one or more Cₙ₊₁ - C₁₁ aromatic hydrocarbons;
(II) the hydroalkylation reaction effluent in step (a) further comprises (iii) one or more C₂ₙ₊₂ - C₂₂ cycloalkylaromatic compounds and (iv) one or more Cₙ₊₁ - C₁₁ saturated cyclic hydrocarbons;
(III) at least a portion of the one or more C₂ₙ₊₂ - C₂₂ cycloalkylaromatic compounds and at least a portion of the one or more Cₙ₊₁ - C₁₁ saturated cyclic hydrocarbons are dehydrogenated in the first dehydrogenation zone in step (b) along with the portion of the C₂ₙ cycloalkylaromatic compounds and the portion of the Cₙ saturated cyclic hydrocarbons, such that the first dehydrogenation reaction product in step (b) further comprises (iv) a mixture of C₂ₙ₊₂ - C₂₂ biphenyl compounds; (v) one or more recovered Cₙ₊₁ - C₁₁ aromatic hydrocarbons; and (vi) unreacted Cₙ₊₁ - C₁₁ saturated cyclic hydrocarbons; and
(IV) the light dehydrogenation stream in step (c) further comprises at least a portion of the one or more recovered Cₙ₊₁ - C₁₁ aromatic hydrocarbons and at least a portion of the unreacted Cₙ₊₁ - C₁₁ saturated cyclic hydrocarbons.

8. The process of any one of the foregoing claims, wherein the at least a portion of the light dehydrogenation stream provided to the second dehydrogenation zone in step (d) comprises less than 1.0 wt% C₂ₙ or higher hydrocarbons.

9. The process of any one of the foregoing claims, wherein the rate of conversion of the unreacted Cₙ saturated cyclic hydrocarbons to the additional recovered Cₙ aromatic hydrocarbons in step (e) is at least 90%.

10. The process of any one of the foregoing claims, wherein either or both of the first and second dehydrogenation catalysts of steps (b) and (e), respectively, comprises an element or compound thereof selected from group 10 of the Periodic Table of Elements.

11. The process of any one of the foregoing claims, wherein the dehydrogenating (e) takes place in the presence of less than 1.0 wt% C₂ₙ or higher hydrocarbons.

12. A process for producing biphenyl compounds, the process comprising:
(a) contacting a hydroalkylation feed comprising at least one Cₙ aromatic hydrocarbon with hydrogen in the presence of a hydroalkylation catalyst under conditions effective to produce a hydroalkylation reaction effluent comprising (i) a C₂ₙ cycloalkylaromatic compound and (ii) a Cₙ saturated cyclic hydrocarbon, wherein n is an integer from 6 to 12;
(b) separating the hydroalkylation reaction effluent into (i) a heavy hydroalkylation effluent rich in the C₂ₙ cycloalkylaromatic compound and (ii) a light hydroalkylation effluent rich in the Cₙ saturated cyclic hydrocarbon;
(c) providing at least a portion of the heavy hydroalkylation effluent to a first dehydrogenation zone and therein dehydrogenating at least a portion of the C₂ₙ cycloalkylaromatic compound in the presence of a first dehydrogenation catalyst under conditions effective to produce a heavy dehydrogenation reaction product comprising a mixture of C₂ₙ biphenyl compounds; and
(d) providing at least a portion of the light hydroalkylation effluent to a second dehydrogenation zone separate from the first dehydrogenation zone, and therein dehydrogenating at least a portion of the Cₙ saturated cyclic hydrocarbon in the presence of a second dehydrogenation catalyst under conditions effective to produce a light dehydrogenation reaction product comprising recovered Cₙ aromatic hydrocarbon.

13. The process of claim 12, further comprising:
(e) recycling at least a portion of the light dehydrogenation reaction product of step (d) such that it forms at least a part of the hydroalkylation feed in step (a).

14. The process of claim 13, wherein recycling at least a portion of the light dehydrogenation reaction product comprises:
(e-1) purifying the light dehydrogenation reaction product to obtain a purified light dehydrogenation reaction product; and
(e-2) providing at least a portion of the purified light dehydrogenation reaction product as at least part of the hydroalkylation feed in step (a).

15. The process of any one of claims 12-14, wherein:
in step (a),
the Cₙ aromatic hydrocarbon is selected from the group consisting of benzene, toluene, ethylbenzene, xylene, and diethylbenzene;
the C₂ₙ cycloalkylaromatic compound is selected from the group consisting of cyclohexylbenzene, (methylcyclohexyl)toluene, (ethylcyclohexyl)ethylbenzene, (dimethylcyclohexyl)xylene, (diethylcyclohexyl)diethylbenzene, and mixtures thereof; and
the Cₙ saturated cyclic hydrocarbon is selected from the group consisting of cyclohexane, methylcyclohexane, dimethylcyclohexane, ethylcyclohexane, diethylcyclohexane, and mixtures thereof;
in step (c), the C₂ₙ biphenyl compounds are each selected from the group consisting of biphenyl, dimethylbiphenyl, diethylbiphenyl, tetramethylbiphenyl, tetraethylbiphenyl, and mixtures thereof; and
in step (d), the recovered Cₙ aromatic hydrocarbon comprises the same compound or compounds as the Cₙ aromatic hydrocarbon.

## Patentansprüche

1. Verfahren zur Produktion von Biphenylverbindungen, bei dem
(a) ein Hydroalkylierungseinsatzmaterial, das aromatische Cₙ-Kohlenwasserstoffe umfasst, in Gegenwart eines Hydroalkylierungskatalysators unter Bedingungen mit Wasserstoff in Kontakt gebracht wird, die effektiv sind, um einen Hydroalkylierungsreaktionsausfluss zu produzieren, der (i) cycloalkylaromatische C₂ₙ-Verbindungen und (ii) gesättigte cyclische Cₙ-Kohlenwasserstoffe umfasst, wobei n eine ganze Zahl von 6 bis 12 ist,
(b) einer ersten Dehydrierungszone mindestens ein Anteil des Hydroalkylierungsreaktionsausflusses bereitgestellt wird, worin mindestens ein Anteil der cycloalkylaromatischen C₂ₙ-Verbindungen und mindestens ein Anteil der gesättigten cyclischen Cₙ-Kohlenwasserstoffe in Gegenwart eines ersten Dehydrierungskatalysators unter Bedingungen dehydriert werden, die effektiv sind, um ein erstes Dehydrierungsreaktionsprodukt zu produzieren, das (i) eine Mischung von C₂ₙ-Biphenylverbindungen, (ii) zurückgewonnene aromatische Cₙ-Kohlenwasserstoffe und (iii) nicht-umgesetzte gesättigte cyclische Cₙ-Kohlenwasserstoffe umfasst,
(c) das erste Dehydrierungsreaktionsprodukt in (i) einen schweren Dehydrierungsstrom, der reich an den C₂ₙ-Biphenylverbindungen ist, und (ii) einen leichten Dehydrierungsstrom getrennt wird, der reich an den zurückgewonnenen aromatischen Cₙ-Kohlenwasserstoffen und den nicht-umgesetzten gesättigten cyclischen Cₙ-Kohlenwasserstoffen ist,
(d) einer zweiten Dehydrierungszone, die getrennt von der ersten Dehydrierungszone vorliegt, mindestens ein Anteil des leichten Dehydrierungsstroms bereitgestellt wird, und
(e) in der zweiten Dehydrierungszone mindestens ein Anteil der nicht-umgesetzten gesättigten cyclischen Cₙ-Kohlenwasserstoffe in Gegenwart eines zweiten Dehydrierungskatalysators unter Bedingungen dehydriert wird, die effektiv sind, um ein zweites Dehydrierungsreaktionsprodukt zu produzieren, das zusätzliche zurückgewonnene aromatische Cₙ-Kohlenwasserstoffe umfasst.

2. Verfahren nach Anspruch 1, bei dem des Weiteren (f) mindestens ein Anteil des zweiten Dehydrierungsreaktionsprodukts recycelt wird, so dass er mindestens einen Teil des Hydroalkylierungseinsatzmaterials für den Hydroalkylierungsschritt (a) bildet.

3. Verfahren nach einem der Ansprüche 1 bis 2, bei dem:
in Schritt (a)
die aromatischen Cₙ-Kohlenwasserstoffe ausgewählt sind aus der Gruppe bestehend aus Benzol, Toluol, Ethylbenzol, Xylol und Diethylbenzol,
die cycloalkylaromatischen C₂ₙ-Verbindungen ausgewählt sind aus der Gruppe bestehend aus Cyclohexylbenzol, (Methylcyclohexyl)toluol, (Ethylcyclohexyl)ethylbenzol, (Dimethylcyclohexyl)xylol und (Diethylcyclohexyl)diethylbenzol, und
die gesättigten cyclischen Cₙ-Kohlenwasserstoffe ausgewählt sind aus der Gruppe bestehend aus Cyclohexan, Methylcyclohexan, Dimethylcyclohexan, Ethylcyclohexan und Diethylcyclohexan,
in Schritt (b)
die C₂ₙ-Biphenylverbindungen jeweils ausgewählt sind aus der Gruppe bestehend aus Biphenyl, Dimethylbiphenyl, Diethylbiphenyl, Tetramethylbiphenyl und Tetraethylbiphenyl,
die zurückgewonnenen aromatischen Cₙ-Kohlenwasserstoffe dieselbe Verbindung oder dieselben Verbindungen wie die aromatischen Cₙ-Kohlenwasserstoffe umfassen, und
die nicht-umgesetzten gesättigten cyclischen Cₙ-Kohlenwasserstoffe dieselbe Verbindung oder dieselben Verbindungen wie die gesättigten cyclischen Cₙ-Kohlenwasserstoffe umfassen, und
in Schritt (e)
die zusätzlichen zurückgewonnenen aromatischen Cₙ-Kohlenwasserstoffe dieselbe Verbindung oder dieselben Verbindungen wie die aromatischen Cₙ-Kohlenwasserstoffe umfassen.

4. Verfahren nach Anspruch 3, bei dem
in Schritt (a) die aromatischen Cₙ-Kohlenwasserstoffe Toluol sind, die cycloalkylaromatischen C₂ₙ-Verbindungen (Methylcyclohexyl)toluol sind, und die gesättigten cyclischen Cₙ-Kohlenwasserstoffe Methylcyclohexan sind,
in Schritt (b) die C₂ₙ-Biphenylverbindungen Dimethylbiphenyl sind, die zurückgewonnenen aromatischen Cₙ-Kohlenwasserstoffe Toluol sind, und die nicht-umgesetzten gesättigten cyclischen Cₙ-Kohlenwasserstoffe Methylcyclohexan sind, und
in Schritt (e) die zusätzlichen zurückgewonnenen aromatischen Cₙ-Kohlenwasserstoffe Toluol sind.

5. Verfahren nach Anspruch 3 oder Anspruch 4, bei dem des Weiteren
(g) mindestens ein Anteil des in Schritt (c) erhaltenen schweren Dehydrierungsstroms unter Bedingungen mit einem Oxidationsmittel in Kontakt gebracht wird, die effektiv sind, um mindestens einen Teil der C₂ₙ-Biphenylverbindungen in Biphenylcarbonsäuren umzuwandeln, und
(h) die Biphenylcarbonsäuren unter Bedingungen mit einem oder mehreren C₁- bis C₁₄-Alkoholen umgesetzt werden, die effektiv sind, um Biphenylester zu produzieren.

6. Verfahren nach einem der Ansprüche 1 bis 2, bei dem n eine ganze Zahl von 7 bis 11 ist, und bei dem des Weiteren
(I) das Hydroalkylierungseinsatzmaterial in Schritt (a) des Weiteren einen oder mehrere aromatische Cₙ₊₁- bis C₁₂-Kohlenwasserstoffe umfasst,
(II) der Hydroalkylierungsreaktionsausfluss in Schritt (a) des Weiteren (iii) eine oder mehrere cycloalkylaromatische C₂ₙ₊₂- bis C₂₄-Verbindungen und (iv) einen oder mehrere gesättigte cyclische Cₙ₊₁- bis C₁₂-Kohlenwasserstoffe umfasst,
(III) mindestens ein Anteil der einen oder mehreren cycloalkylaromatischen C₂ₙ₊₂- bis C₂₄-Verbindungen und mindestens ein Anteil des einen oder der mehreren gesättigten cyclischen Cₙ₊₁- bis C₁₂-Kohlenwasserstoffe in Schritt (b) zusammen mit dem Anteil der cycloalkylaromatischen C₂ₙ-Verbindungen und dem Anteil der gesättigten cyclischen Cₙ-Kohlenwasserstoffe in der ersten Dehydrierungszone dehydriert werden, so dass das erste Dehydrierungsreaktionsprodukt in Schritt (b) des Weiteren (iv) eine Mischung aus C₂ₙ₊₂- bis C₂₄-Biphenylverbindungen, (v) einen oder mehrere zurückgewonnene aromatische Cₙ₊₁- bis C₁₂-Kohlenwasserstoffe und (vi) nicht-umgesetzte gesättigte cyclische Cₙ₊₁- bis C₁₂-Kohlenwasserstoffe umfasst, und
(IV) der leichte Dehydrierungsstrom in Schritt (c) des Weiteren mindestens einen Anteil des einen oder der mehreren zurückgewonnenen aromatischen Cₙ₊₁- bis C₁₂-Kohlenwasserstoffe und mindestens einen Anteil der nicht-umgesetzten gesättigten cyclischen Cₙ₊₁- bis C₁₂-Kohlenwasserstoffe umfasst.

7. Verfahren nach einem der Ansprüche 1 bis 2, bei dem n eine ganze Zahl von 6 bis 10 ist, und bei dem des Weiteren
(I) das Hydroalkylierungseinsatzmaterial in Schritt (a) des Weiteren einen oder mehrere aromatische Cₙ₊₁- bis C₁₁-Kohlenwasserstoffe umfasst,
(II) der Hydroalkylierungsreaktionsausfluss in Schritt (a) des Weiteren (iii) eine oder mehrere cycloalkylaromatische C₂ₙ₊₂- bis C₂₂-Verbindungen und (iv) einen oder mehrere gesättigte cyclische Cₙ₊₁- bis C₁₁-Kohlenwasserstoffe umfasst,
(III) mindestens ein Anteil des einen oder der mehreren cycloalkylaromatischen C₂ₙ₊₂- bis C₂₂-Verbindungen und mindestens ein Anteil der einen oder mehreren gesättigten cyclischen Cₙ₊₁- bis C₁₁-Kohlenwasserstoffe in Schritt (b) zusammen mit dem Anteil der cycloalkylaromatischen C₂ₙ-Verbindungen und dem Anteil der gesättigten cyclischen Cₙ-Kohlenwasserstoffe in der ersten Dehydrierungszone dehydriert werden, so dass das erste Dehydrierungsreaktionsprodukt in Schritt (b) des Weiteren (iv) eine Mischung aus C₂ₙ₊₂- bis C₂₂-Biphenylverbindungen, (v) einen oder mehrere zurückgewonnene aromatische Cₙ₊₁- bis C₁₁-Kohlenwasserstoffe und (vi) nicht-umgesetzte gesättigte cyclische Cₙ₊₁- bis C₁₁-Kohlenwasserstoffe umfasst, und
(IV) der leichte Dehydrierungsstrom in Schritt (c) des Weiteren mindestens einen Anteil des einen oder der mehreren zurückgewonnenen aromatischen Cₙ₊₁- bis Cn-Kohlenwasserstoffe und mindestens einen Anteil der nicht-umgesetzten gesättigten cyclischen Cₙ₊₁- bis C₁₁-Kohlenwasserstoffe umfasst.

8. Verfahren nach einem der vorhergehenden Ansprüche, bei dem der mindestens eine Anteil des leichten Dehydrierungsstroms, der der zweiten Dehydrierungszone in Schritt (d) bereitgestellt wird, weniger als 1,0 Gew.% C₂ₙ- oder höhere Kohlenwasserstoffe umfasst.

9. Verfahren nach einem der vorhergehenden Ansprüche, bei dem die Umwandlungsrate der nicht-umgesetzten gesättigten cyclischen Cₙ-Kohlenwasserstoffe zu den zusätzlichen zurückgewonnenen aromatischen Cₙ-Kohlenwasserstoffen in Schritt (e) mindestens 90 % beträgt.

10. Verfahren nach einem der vorhergehenden Ansprüche, bei dem einer oder beide von dem ersten und dem zweiten Dehydrierungskatalysator in Schritt (b) beziehungsweise (e) ein Element ausgewählt aus Gruppe 10 des Periodensystems der Elemente oder eine Verbindung davon umfasst.

11. Verfahren nach einem der vorhergehenden Ansprüche, bei dem die Dehydrierung (e) in Gegenwart von weniger als 1, 0 Gew. % C₂ₙ- oder höheren Kohlenwasserstoffen stattfindet.

12. Verfahren zur Produktion von Biphenylverbindungen, bei dem
(a) ein Hydroalkylierungseinsatzmaterial, das mindestens einen aromatischen Cₙ-Kohlenwasserstoff umfasst, in Gegenwart eines Hydroalkylierungskatalysators unter Bedingungen mit Wasserstoff in Kontakt gebracht wird, die effektiv sind, um einen Hydroalkylierungsreaktionsausfluss zu produzieren, der (i) cycloalkylaromatische C₂ₙ-Verbindung und (ii) gesättigten cyclischen Cₙ-Kohlenwasserstoff umfasst, wobei n eine ganze Zahl von 6 bis 12 ist,
(b) der Hydroalkylierungsreaktionsausfluss in (i) einen schweren Hydroalkylierungsausfluss, der reich an der cycloalkylaromatischen C₂ₙ-Verbindung ist, und (ii) einen leichten Hydroalkylierungsausfluss getrennt wird, der reich an der gesättigten cyclischen Cₙ-Verbindung ist,
(c) mindestens ein Anteil des schweren Hydroalkylierungsausflusses einer ersten Dehydrierungszone bereitgestellt wird, und darin mindestens ein Anteil der cycloalkylaromatischen C₂ₙ-Verbindung in Gegenwart eines ersten Dehydrierungskatalysators unter Bedingungen dehydriert wird, die effektiv sind, um ein schweres Dehydrierungsreaktionsprodukt zu produzieren, das eine Mischung von C₂ₙ-Biphenylverbindungen umfasst, und
(d) mindestens ein Anteil des leichten Hydroalkylierungsausflusses einer zweiten Dehydrierungszone bereitgestellt wird, die getrennt von der ersten Dehydrierungszone vorliegt, und in der mindestens ein Anteil des gesättigten cyclischen Cₙ-Kohlenwasserstoffs in Gegenwart eines zweiten Dehydrierungskatalysators unter Bedingungen dehydriert wird, die effektiv sind, um ein leichtes Dehydrierungsreaktionsprodukt zu produzieren, das zurückgewonnenen aromatischen Cₙ-Kohlenwasserstoff umfasst.

13. Verfahren nach Anspruch 12, bei dem des Weiteren
(e) mindestens ein Anteil des leichten Dehydrierungsreaktionsprodukts aus Schritt (d) recycelt wird, so dass es mindestens einen Teil des Hydroalkylierungseinsatzmaterials in Schritt (a) bildet.

14. Verfahren nach Anspruch 13, bei dem Recyceln von mindestens einem Anteil des leichten Dehydrierungsreaktionsprodukts umfasst:
(e-1) Reinigen des leichten Dehydrierungsreaktionsprodukts, um ein gereinigtes leichtes Dehydrierungsreaktionsprodukt zu erhalten, und
(e-2) Bereitstellen mindestens eines Anteils des gereinigten leichten Dehydrierungsreaktionsprodukts als mindestens Teil des Hydroalkylierungseinsatzmaterials in Schritt (a).

15. Verfahren nach einem der Ansprüche 12 bis 14, bei dem in Schritt (a)
der aromatische Cₙ-Kohlenwasserstoff ausgewählt ist aus der Gruppe bestehend aus Benzol, Toluol, Ethylbenzol, Xylol und Diethylbenzol,
die cycloalkylaromatische C₂ₙ-Verbindung ausgewählt ist aus der Gruppe bestehend aus Cyclohexylbenzol, (Methylcyclohexyl)-toluol, (Ethylcyclohexyl)ethylbenzol, (Dimethylcyclohexyl)-xylol, (Diethylcyclohexyl) diethylbenzol und Mischungen davon, und
der gesättigte cyclische Cₙ-Kohlenwasserstoff ausgewählt ist aus der Gruppe bestehend aus Cyclohexan, Methylcyclohexan, Dimethylcyclohexan, Ethylcyclohexan, Diethylcyclohexan und Mischungen davon,
in Schritt (c) die C₂ₙ-Biphenylverbindungen jeweils ausgewählt sind aus der Gruppe bestehend aus Biphenyl, Dimethylbiphenyl, Diethylbiphenyl, Tetramethylbiphenyl, Tetraethylbiphenyl und Mischungen davon, und
in Schritt (d) der zurückgewonnene aromatische Cₙ-Kohlenwasserstoff dieselbe Verbindung oder dieselben Verbindungen wie der aromatische Cₙ-Kohlenwasserstoff umfasst.

## Revendications

1. Procédé de production de composés biphényliques, le procédé comprenant :
(a) la mise en contact d'une charge d'hydroalkylation comprenant des hydrocarbures aromatiques en Cₙ avec de l'hydrogène en présence d'un catalyseur d'hydroalkylation dans des conditions efficaces pour produire un effluent de réaction d'hydroalkylation comprenant (i) des composés cycloalkylaromatiques en C₂ₙ et (ii) des hydrocarbures cycliques saturés en Cₙ, où n est un entier de 6 à 12 ;
(b) le transfert d'au moins une partie de l'effluent de réaction d'hydroalkylation vers une première zone de déshydrogénation, dans laquelle est conduite la déshydrogénation d'au moins une partie des composés cycloalkylaromatiques en C₂ₙ et d'au moins une partie des hydrocarbures cycliques saturés en Cₙ en présence d'un premier catalyseur de déshydrogénation dans des conditions efficaces pour produire un premier produit de réaction de déshydrogénation comprenant (i) un mélange de composés biphényliques en C₂ₙ ; (ii) des hydrocarbures aromatiques en Cₙ récupérés ; et (iii) des hydrocarbures cycliques saturés en Cₙ n'ayant pas réagi ;
(c) la séparation du premier produit de réaction de déshydrogénation en (i) un flux de déshydrogénation lourd riche en composés biphényliques en C₂ₙ, et (ii) un flux de déshydrogénation léger riche en hydrocarbures aromatiques en Cₙ récupérés et en hydrocarbures cycliques saturés en Cₙ n'ayant pas réagi ;
(d) le transfert d'au moins une partie du flux de déshydrogénation léger vers une deuxième zone de déshydrogénation séparée de la première zone de déshydrogénation ; et
(e) dans la deuxième zone de déshydrogénation, la déshydrogénation d'au moins une partie des hydrocarbures cycliques saturés en Cₙ n'ayant pas réagi en présence d'un deuxième catalyseur de déshydrogénation dans des conditions efficaces pour produire un deuxième produit de réaction de déshydrogénation comprenant des hydrocarbures aromatiques en Cₙ récupérés supplémentaires.

2. Procédé selon la revendication 1, comprenant en outre (f) le recyclage d'au moins une partie du deuxième produit de réaction de déshydrogénation de sorte qu'il forme au moins une partie de la charge d'hydroalkylation pour l'étape d'hydroalkylation (a).

3. Procédé selon l'une quelconque des revendications 1 à 2, dans lequel :
dans l'étape (a),
les hydrocarbures aromatiques en Cₙ sont choisis dans le groupe constitué des benzène, toluène, éthylbenzène, xylène et diéthylbenzène ;
les composés cycloalkylaromatiques en C₂ₙ sont choisis dans le groupe constitué des cyclohexylbenzène, (méthylcyclohexyl)toluène, (éthylcyclohexyl)éthylbenzène, (diméthylcyclohexyl)xylène et (diéthylcyclohexyl)diéthylbenzène ; et
les hydrocarbures cycliques saturés en Cₙ sont choisis dans le groupe constitué des cyclohexane, méthylcyclohexane, diméthylcyclohexane, éthylcyclohexane et diéthylcyclohexane ;
dans l'étape (b),
les composés biphényliques en C₂ₙ sont chacun choisis dans le groupe constitué des biphényle, diméthylbiphényle, diéthylbiphényle, tétraméthylbiphényle et tétraéthylbiphényle ; les hydrocarbures aromatiques en Cₙ récupérés comprennent le même composé ou les mêmes composés que les hydrocarbures aromatiques en Cₙ ; et
les hydrocarbures cycliques saturés en Cₙ n'ayant pas réagi comprennent le même composé ou les mêmes composés que les hydrocarbures cycliques saturés en Cₙ ; et
dans l'étape (e),
les hydrocarbures aromatiques en Cₙ récupérés supplémentaires comprennent le même composé ou les mêmes composés que les hydrocarbures aromatiques en Cₙ.

4. Procédé selon la revendication 3, dans lequel :
dans l'étape (a), les hydrocarbures aromatiques en Cₙ sont le toluène, les composés cycloalkylaromatiques en C₂ₙ sont le (méthylcyclohexyl)toluène, et les hydrocarbures cycliques saturés en Cₙ sont le méthylcyclohexane ;
dans l'étape (b), les composés biphényliques en C₂ₙ sont le diméthylbiphényle, les hydrocarbures aromatiques en Cₙ récupérés sont le toluène, et les hydrocarbures cycliques saturés en Cₙ n'ayant pas réagi sont le méthylcyclohexane ; et
dans l'étape (e), les hydrocarbures aromatiques en Cₙ récupérés supplémentaires sont le toluène.

5. Procédé selon la revendication 3 ou la revendication 4, comprenant en outre :
(g) la mise en contact d'au moins une partie du flux de déshydrogénation lourd obtenu dans l'étape (c) avec un oxydant dans des conditions efficaces pour convertir au moins une partie des composés biphényliques en C₂ₙ en acides biphénylcarboxyliques ; et
(h) la réaction des acides biphénylcarboxyliques avec un ou plusieurs alcools en C₁ à C₁₄ dans des conditions efficaces pour produire des esters biphényliques.

6. Procédé selon l'une quelconque des revendications 1 à 2, dans lequel n est un entier de 7 à 11, et en outre dans lequel :
(I) la charge d'hydroalkylation dans l'étape (a) comprend en outre un ou plusieurs hydrocarbures aromatiques en Cₙ₊₁ à C₁₂ ;
(II) l'effluent de réaction d'hydroalkylation dans l'étape (a) comprend en outre (iii) un ou plusieurs composés cycloalkylaromatiques en C₂ₙ₊₂ à C₂₄ et (iv) un ou plusieurs hydrocarbures cycliques saturés en Cₙ₊₁ à C₁₂ ;
(III) au moins une partie des un ou plusieurs composés cycloalkylaromatiques en C₂ₙ₊₂ à C₂₄ et au moins une partie des un ou plusieurs hydrocarbures cycliques saturés en Cₙ₊₁ à C₁₂ sont déshydrogénés dans la première zone de déshydrogénation dans l'étape (b) conjointement avec la partie des composés cycloalkylaromatiques en C₂ₙ et la partie des hydrocarbures cycliques saturés en Cₙ, de sorte que le premier produit de réaction de déshydrogénation dans l'étape (b) comprenne en outre (iv) un mélange de composés biphényliques en C₂ₙ₊₂ à C₂₄ ; (v) un ou plusieurs hydrocarbures aromatiques en Cₙ₊₁ à C₁₂ récupérés ; et (vi) des hydrocarbures cycliques saturés en Cₙ₊₁ à C₁₂ n'ayant pas réagi ; et
(IV) le flux de déshydrogénation léger dans l'étape (c) comprend en outre au moins une partie des un ou plusieurs hydrocarbures aromatiques en Cₙ₊₁ à C₁₂ récupérés et au moins une partie des hydrocarbures cycliques saturés en Cₙ₊₁ à C₁₂ n'ayant pas réagi.

7. Procédé selon l'une quelconque des revendications 1 à 2, dans lequel n est un entier de 6 à 10, et en outre dans lequel :
(I) la charge d'hydroalkylation dans l'étape (a) comprend en outre un ou plusieurs hydrocarbures aromatiques en Cₙ₊₁ à C₁₁ ;
(II) l'effluent de réaction d'hydroalkylation dans l'étape (a) comprend en outre (iii) un ou plusieurs composés cycloalkylaromatiques en C₂ₙ₊₂ à C₂₂ et (iv) un ou plusieurs hydrocarbures cycliques saturés en Cₙ₊₁ à C₁₁ ;
(III) au moins une partie des un ou plusieurs composés cycloalkylaromatiques en C₂ₙ₊₂ à C₂₂ et au moins une partie des un ou plusieurs hydrocarbures cycliques saturés en Cₙ₊₁ à C₁₁ sont déshydrogénés dans la première zone de déshydrogénation dans l'étape (b) conjointement avec la partie des composés cycloalkylaromatiques en C₂ₙ et la partie des hydrocarbures cycliques saturés en Cₙ, de sorte que le premier produit de réaction de déshydrogénation dans l'étape (b) comprenne en outre (iv) un mélange de composés biphényliques en C₂ₙ₊₂ à C₂₂ ; (v) un ou plusieurs hydrocarbures aromatiques en Cₙ₊₁ à C₁₁ récupérés ; et (vi) des hydrocarbures cycliques saturés en Cₙ₊₁ à C₁₁ n' ayant pas réagi ; et
(IV) le flux de déshydrogénation léger dans l'étape (c) comprend en outre au moins une partie des un ou plusieurs hydrocarbures aromatiques en Cₙ₊₁ à C₁₁ récupérés et au moins une partie des hydrocarbures cycliques saturés en Cₙ₊₁ à C₁₁ n'ayant pas réagi.

8. Procédé selon l'une quelconque des revendications précédentes, dans lequel l'au moins une partie du flux de déshydrogénation léger transférée vers la deuxième zone de déshydrogénation dans l'étape (d) comprend moins de 1,0 % en poids d'hydrocarbures en C₂ₙ ou supérieurs.

9. Procédé selon l'une quelconque des revendications précédentes, dans lequel le taux de conversion des hydrocarbures cycliques saturés en Cₙ n'ayant pas réagi en hydrocarbures aromatiques en Cₙ récupérés supplémentaires dans l'étape (e) est au moins 90 %.

10. Procédé selon l'une quelconque des revendications précédentes, dans lequel l'un ou les deux parmi les premier et deuxième catalyseurs de déshydrogénation des étapes (b) et (e), respectivement, comprend un élément ou un composé de celui-ci choisi parmi le groupe 10 de la table périodique des éléments.

11. Procédé selon l'une quelconque des revendications précédentes, dans lequel la déshydrogénation (e) est conduite en présence de moins de 1,0 % en poids d'hydrocarbures en C₂ₙ ou supérieurs.

12. Procédé de production de composés biphényliques, le procédé comprenant :
(a) la mise en contact d'une charge d'hydroalkylation comprenant au moins un hydrocarbure aromatique en Cₙ avec de l'hydrogène en présence d'un catalyseur d'hydroalkylation dans des conditions efficaces pour produire un effluent de réaction d'hydroalkylation comprenant (i) un composé cycloalkylaromatique en C₂ₙ et (ii) un hydrocarbure cyclique saturé en Cₙ, dans lequel n est un entier de 6 à 12 ;
(b) la séparation de l'effluent de réaction d'hydroalkylation en (i) un effluent d'hydroalkylation lourd riche en composé cycloalkylaromatique en C₂ₙ et (ii) un effluent d'hydroalkylation léger riche en hydrocarbure cyclique saturé en Cₙ ;
(c) le transfert d'au moins une partie de l'effluent d'hydroalkylation lourd vers une première zone de déshydrogénation et dans laquelle est conduite la déshydrogénation d'au moins une partie du composé cycloalkylaromatique en C₂ₙ en présence d'un premier catalyseur de déshydrogénation dans des conditions efficaces pour produire un produit de réaction de déshydrogénation lourd comprenant un mélange de composés biphényliques en C₂ₙ ; et
(d) le transfert d'au moins une partie de l'effluent d'hydroalkylation léger vers une deuxième zone de déshydrogénation séparée de la première zone de déshydrogénation, et dans laquelle est conduite la déshydrogénation d'au moins une partie de l'hydrocarbure cyclique saturé en Cₙ en présence d'un deuxième catalyseur de déshydrogénation dans des conditions efficaces pour produire un produit de réaction de déshydrogénation léger comprenant un hydrocarbure aromatique en Cₙ récupéré.

13. Procédé selon la revendication 12, comprenant en outre :
(e) le recyclage d'au moins une partie du produit de réaction de déshydrogénation léger de l'étape (d) de sorte qu'il forme au moins une partie de la charge d'hydroalkylation dans l'étape (a) .

14. Procédé selon la revendication 13, dans lequel le recyclage d'au moins une partie du produit de réaction de déshydrogénation léger comprend :
(e-1) la purification du produit de réaction de déshydrogénation léger pour obtenir un produit de réaction de déshydrogénation léger purifié ; et
(e-2) le transfert d'au moins une partie du produit de réaction de déshydrogénation léger purifié en tant qu'au moins une partie de la charge d'hydroalkylation dans l'étape (a).

15. Procédé selon l'une quelconque des revendications 12 à 14, dans lequel :
dans l'étape (a),
l'hydrocarbure aromatique en Cₙ est choisi dans le groupe constitué des benzène, toluène, éthylbenzène, xylène et diéthylbenzène ;
le composé cycloalkylaromatique en C₂ₙ est choisi dans le groupe constitué des cyclohexylbenzène, (méthylcyclohexyl)toluène, (éthylcyclohexyl)éthylbenzène, (diméthylcyclohexyl)xylène, (diéthylcyclohexyl)diéthylbenzène, et des mélanges de ceux-ci ; et
l'hydrocarbure cyclique saturé en Cₙ est choisi dans le groupe constitué des cyclohexane, méthylcyclohexane, diméthylcyclohexane, éthylcyclohexane, diéthylcyclohexane, et
des mélanges de ceux-ci ;
dans l'étape (c), les composés biphényliques en C₂ₙ sont chacun choisis dans le groupe constitué des biphényle, diméthylbiphényle, diéthylbiphényle, tétraméthylbiphényle, tétraéthylbiphényle, et des mélanges de ceux-ci ; et
dans l'étape (d), l'hydrocarbure aromatique en Cₙ récupéré comprend le même composé ou les mêmes composés que l'hydrocarbure aromatique en Cₙ.
